# FASCICULE DE BREVET EUROPEEN

(11) **EP 0 634 396 B1**
(45) Date de publication et mention de la délivrance du brevet: **28.10.1998**
(21) Numéro de dépôt: 94401632.8
(22) Date de dépôt: 13.07.1994
(51) Int. Cl.: C07C 327/32, C07C 323/60, C07D 317/60, A61K 31/265, A61K 31/16

(54) **Dérivés d'amino-acides et leur utilisation comme inhibiteurs de l'enképhalinase**
Aminosäurederivate und ihre Verwendung als Enkephalinase-Inhibitoren
Amino acid derivatives and their use as inhibitors of encephalinase

(30) Priorité: 16.07.1993 FR 9308765
(43) Date de publication de la demande: 18.01.1995
(73) Titulaire: SOCIETE CIVILE BIOPROJET, F-75003 Paris (FR)
(72) Inventeur: Danvy, Denis, F-76190 Yvetot (FR); Monteil, Thierry, F-76130 Mont-Saint-Aignan (FR); Lusson, Christophe, F-76000 Rouen (FR); Schwartz, Jean-Charles, F-75014 Paris (FR); Gros, Claude, F-75015 Paris (FR); Noel, Nadine, F-75015 Paris (FR); Lecomte, Jeanne-Marie, F-75003 Paris (FR); Duhamel, Pierre, F-76130 Mont-Saint-Aignan (FR); Duhamel, Lucette, F-76130 Mont-Saint-Aignan (FR)
(74) Mandataire: Bernasconi, Jean

(56) Documents cités:
- EP-A- 0 136 883
- EP-A- 0 419 327
- FR-A- 2 556 721

## Description

La présente invention a trait à de nouveaux dérivés d'amino-acides, à leurs procédés de préparation et à leur application thérapeutique.

Les nouveaux dérivés selon l'invention sont des inhibiteurs de l'enképhalinase qui est une enzyme dégradant les enképhalines.

La méthionine-enképhaline Met-E (Tyr-Gly-Gly-Phe-Met) et la leucine enképhaline Leu-E (Tyr-Gly-Gly-Phe-Leu) sont des pentapeptides qui ont été découverts dans le cerveau et qui sont des ligands endogènes du récepteur morphinique (J. Hughes et al., Nature, 258, 577-579 (1975)).

Ces peptides sont considérés comme des neuromédiateurs à action inhibitrice sur la libération d'autres neurotransmetteurs (J. Hughes, Nature 278, 394 (1979) ; S.H. Snyder, Nature, 278, 13 (1979)). Il a été démontré que les enképhalines sont rapidement dégradées par une carboxydipeptidase ("enképhalinase") qui libère les restes Tyr-Gly-Gly et Phe-Met (B. Malfroy et al., Nature, 276, p. 523-526 (1978)).

Les composés susceptibles d'inhiber l'enképhalinase peuvent donc prolonger les effets des enképhalines endogènes ou potentialiser l'action d'analogues synthétiques administrés de façon exogène. Ces composés sont donc susceptibles de remplacer les agents morphiniques dans toutes leurs propriétés sans en avoir les inconvénients, en particulier au niveau des phénomènes d'accoutumance et de dépendance.

La mise au point des composés susceptibles d'inhiber la dégradation des enképhalines par l'enzyme enképhalinase a fait l'objet de nombreuses recherches.

A titre d'illustration de l'état de la technique, on peut citer les demandes de brevet européen 0 038 758 et 0 082 088 (Roques et al.) qui décrivent des dérivés d'acides aminés et plus particulièrement, des dérivés mercaptoalcanoyle et acylmercaptoalcanoyle d'acides aminés présentant une activité inhibitrice de l'enképhalinase.

Le brevet US n° 4 401 677 (Greenberg et al.) décrit divers mercaptoalcanoyle amino-acides qui sont utiles comme agents analgésiques, en raison de leur activité inhibitrice de l'enképhalinase.

On peut encore mentionner la demande de brevet européen n° 0 136 883 (Delaney et al.) qui décrit des inhibiteurs de l'enképhalinase répondant à la formule générale 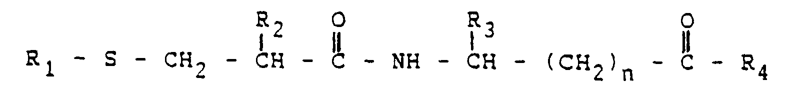 dans laquelle R₁ représente un atome d'hydrogène ou un groupe acyle, R₂ représente, entre autres, un radical benzyle, le noyau benzénique étant éventuellement mono- ou polysubstitué par un atome d'halogène, un groupe trifluorométhyle, un groupe nitro, un groupe alkyle inférieur, un groupe alcoxy inférieur, R₃ représente, entre autres, un atome d'hydrogène, un alkyle inférieur, R₄ représente un hydroxy, des esters ou amides et n est un nombre compris entre 1 et 15.

Les nouveaux inhibiteurs d'enképhalinase conformes à l'invention présentent l'avantage par rapport aux inhibiteurs d'enképhalinase antérieurement connus, tels que ceux décrits dans les brevets ou demandes de brevet précités, d'une moindre liaison aux protéines plasmatiques et donc d'une plus grande activité, grâce en particulier à la présence dans leur structure d'une double liaison éthylénique au niveau du carbone en alpha du groupe carbonyle de la fonction amide.

Les nouveaux dérivés d'amino-acides conformes à l'invention répondent aux formules générales 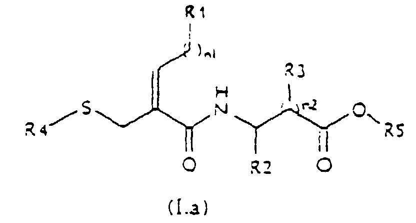 et 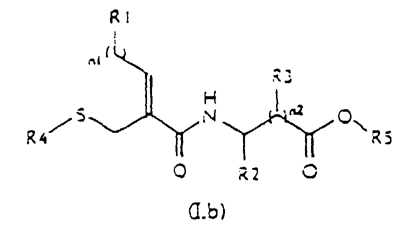 dans lesquelles R₁ représente un atome d'hydrogène ; un groupe phényle, éventuellement mono- ou polysubstitué par un atome d'halogène, un groupe trifluorométhyle, un groupe nitro, un groupe cyano ou un groupe amino, un groupe alkyle inférieur, un groupe phénylalkylène inférieur ;
le groupement 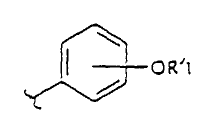 où R' ₁ représente un atome d'hydrogène, un groupe alkyle inférieur, un groupe phényle, un groupe phénylalkylène inférieur ; un groupement 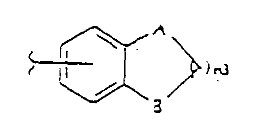 où A, B et n₃ ont la signification donnée ci-dessous

| A | B | n₃ |
|---|---|---|
| 0 | 0 | 1 |
| 0 | CH₂ | 1 |
| CH₂ | CH₂ | 1 |
| 0 | 0 | 2 |
| CH₂ | CH₂ | 2 |
| 0 | CH₂ | 2 |

un groupe biphényle, alpha et béta naphtyle,
n₁ varie de 0 à 10
n₂ varie de 1 à 10
R₂ représente un atome d'hydrogène ; un groupe alkyle inférieur ; un groupe hydroxyalkylène inférieur ; un groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inférieur ; un groupe alkyle inférieur thioalkylène inférieur ; un groupe imidazolylalkylène inférieur ; un groupe indolylalkylène inférieur ; un groupe carbamylalkylène inférieur ; un groupe carboxyalkylène inférieur ;
R₃ représente également un atome d'hydrogène ou un groupe alkyle inférieur.
R₄ représente un atome d'hydrogène, un radical acyle aliphatique linéaire ou ramifié, un radical acyle aromatique éventuellement mono- ou polysubstitué ou un radical acyle linéaire ou ramifié comportant un ou plusieurs atomes d'oxygène ;
R₅ représente un atome d'hydrogène un groupe alkyle inférieur linéaire ou ramifié ; un groupe phényle, un groupe phénylalkylène inférieur, ces deux derniers groupes étant éventuellement mono- ou polysubstitués sur le noyau phényle ; un substituant linéaire ou ramifié comportant un ou plusieurs atomes d'oxygène.

Par groupes alkyles inférieurs, on entend des groupes alkyles à chaîne linéaire ou ramifiée contenant 1 à 6 atomes de carbone et, de préférence, 1 à 4 atomes de carbone.

Par groupes alkylènes inférieurs, on entend des groupes alkylènes contenant 1 à 6 atomes de carbone et, de préférence, 1 à 4 atomes de carbone.

A titre d'atome d'halogène, on préfère particulièrement le fluor.

Comme groupe acyle aliphatique, on peut mentionner les groupes acétyle, propionyle et pivaloyle, le groupe acétyle étant préféré. Comme groupe acyle aromatique, on peut mentionner le groupe benzoyle.

Parmi les composés de formules (Ia) et (Ib) définis ci-dessus, une classe préférée de composés comprend les dérivés dans lesquels :
R₁ représente un groupe phényle éventuellement mono- ou polysubstitué par un atome d'halogène, par le groupe trifluorométhyle, le groupement 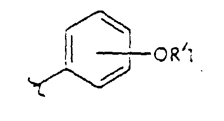 où R'₁ représente un groupe alkyle inférieur, un groupe phényle ; le groupement 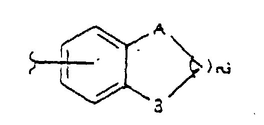 où A et B représentent l'oxygène et n₃ est égal à 1 ou 2 ; un groupe biphényle, alpha et bêta naphtyle ;
R₂ et R₃ représentent un atome d'hydrogène, un radical alkyle inférieur ;
R₄ représente un atome d'hydrogène, un radical acyle aliphatique linéaire ou ramifié ou un radical acyle aromatique ;
R₅ représente un atome d'hydrogène, un radical alkyle inférieur, un radical phényle ;
n₁ est égal à 0 ou 1 ;
n₂ est égal à 1 ou 2.

On préfère, parmi les composés de formules (Ia) et (Ib), ceux qui décrivent les structures amino-acides suivantes : la bêta alanine et l'acide gamma-aminobutyrique.

Parmi les composés de formules (Ia) et (Ib) plus particulièrement préférés, on peut citer (dans les formules données ci-dessous, on désigne par Ph le groupe phényle) :
1) le N-(E)-[1-oxo 2-(acétylthiométhyl) 3-phényl propényl]-β-alaninate de benzyle, 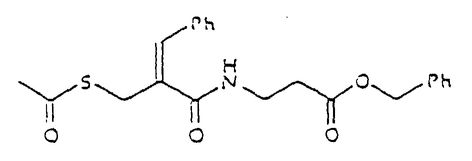
2) le N-(E)-[1-oxo 2-(acétylthiométhyl) 3-phényl propényl]-β-alaninate de méthyle, 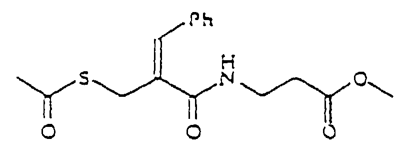
3) le N-(E)-[1-oxo 2-(acétylthiométhyl) 3-phényl propényl]-β-alaninate d'éthyle, 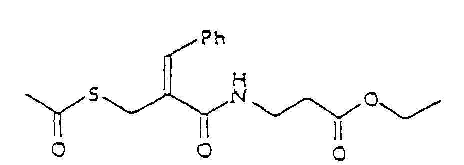
4) la N-(E)-[1-oxo 2-(mercaptométhyl) 3-phényl propényl]-β-alanine, 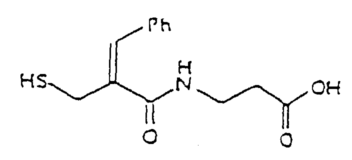
5) le N-(E)-[1-oxo 2-(benzoylthiométhyl) 3-phényl propényl]-β-alaninate de benzyle, 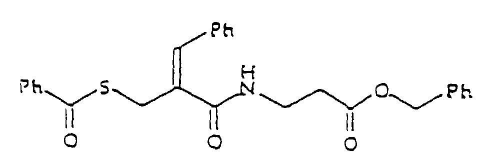
6) le N-(E)-[1-oxo 2-(benzoylthiométhyl) 3-phényl propényl]-β-alaninate de méthyle, 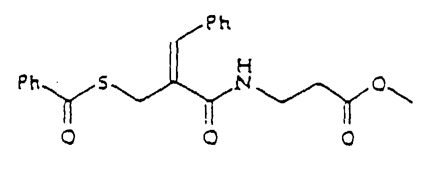
7) le N-(E)-[1-oxo 2-(benzoylthiométhyl) 3-phényl propényl]-β-alaninate d'éthyle, 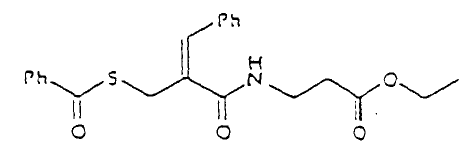
8) le N-(E)-[1-oxo 2-(pivaloylthiométhyl) 3-phényl propényl]-β-alaninate de benzyle, 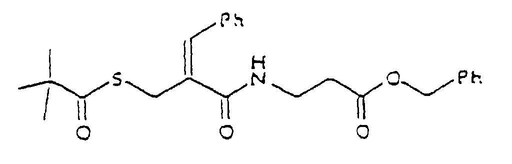
9) le N-(E)-[1-oxo 2-(pivaloylthiométhyl) 3-phényl propényl]-β-alaninate de méthyle, 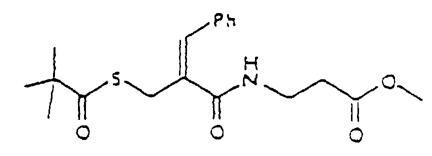
10) le N-(E)-[1-oxo 2-(pivaloylthiométhyl) 3-phényl propényl]-β-alaninate d'éthyle, 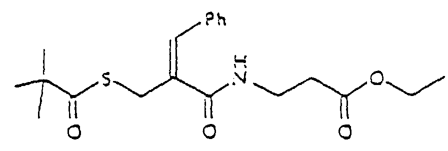
11) le N-(E)-[1-oxo 2-(acétylthiométhyl) 3-phényl propényl]-(RS)-3-amino butanoate de méthyle, 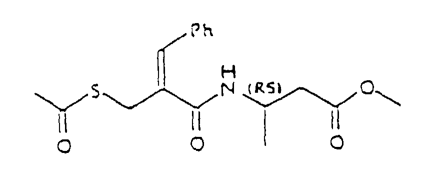
12) l'acide N-(E)-[1-oxo 2-(mercaptométhyl) 3-phényl propényl]-(RS)-3-amino butanoïque, 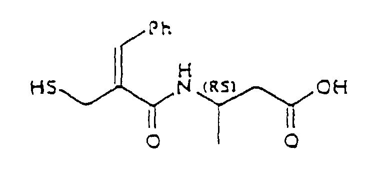
13) le N-(E)-[1-oxo 2-(acétylthiométhyl) 3-phényl propényl]-(RS)-3-amino 2-méthyl propanoate de méthyle, 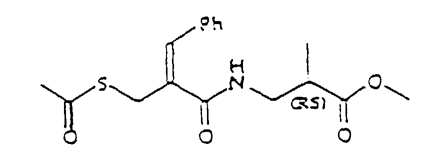
14) l'acide N-(E)-[1-oxo 2-(mercaptométhyl) 3-phényl propényl]-(RS)-3-amino 2-méthyl propanoïque, 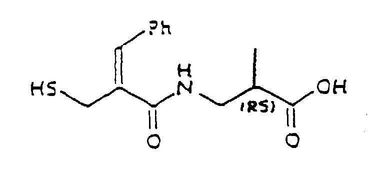
15) le N-(E)-[1-oxo 2-(acétylthiométhyl) 3-phényl propényl]-4-amino butanoate de benzyle, 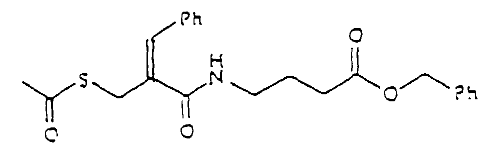
16) l'acide N-(E)-[1-oxo 2-(mercaptométhyl) 3-phényl propényl]-4-amino butanoïque, 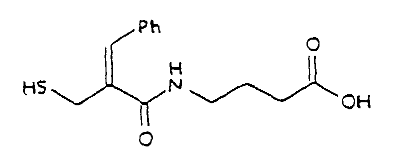
17) le N-(Z)-[1-oxo 2-(acétylthiométhyl) 3-(4-méthoxy phényl)propényl]-β-alaninate de benzyle, 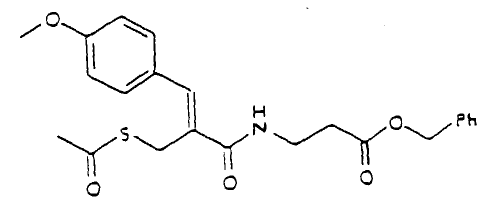
18) le N-(E)-[1-oxo 2-(acétylthiométhyl) 3-(4-méthoxy phényl) propényl]-β-alaninate de benzyle, 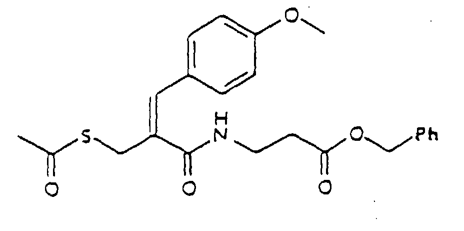
19) le N-(E)-[1-oxo 2-(mercaptométhyl)3-(4-méthoxy phényl) propényl]-β-alanine, 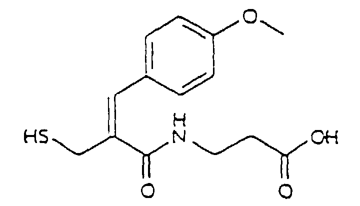
20) le N-(Z)-[1-oxo 2-(acétylthiométhyl) 3-(4-trifluorométhyl phényl) propényl]-β-alaninate de benzyle, 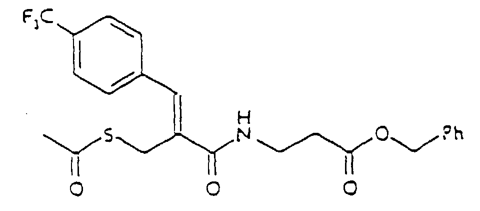
21) le N-(E)-[1-oxo 2-(acétylthiométhyl) 3-(4-trifluorométhyl phényl) propényl]-β-alaninate de benzyle, 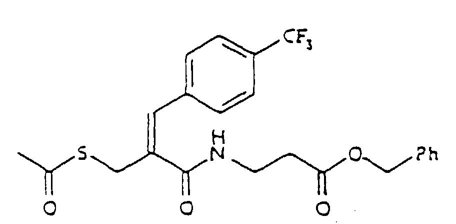
22) la N-(E)-[1-oxo 2-(mercaptométhyl) 3-(4-trifluorométhyl phényl) propényl]-β-alanine, 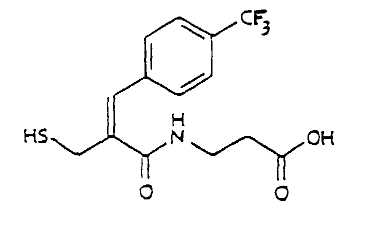
23) le N-(Z)-[1-oxo 2-(acétylthiométhyl) 3-(4-phényl phényl)propényl]-β-alaninate de benzyle, 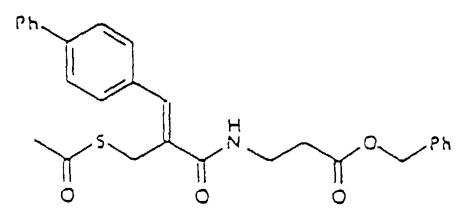
24) le N-(E)-[1-oxo 2-(acétylthiométhyl) 3-(4-phényl phényl) propényl]-β-alaninate de benzyle, 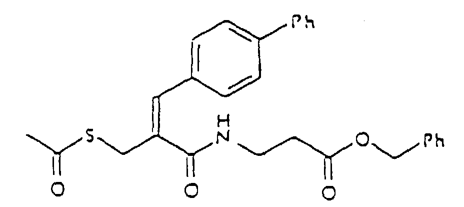
25) la N-(E)-[1-oxo 2-(mercaptométhyl) 3-(4-phényl phényl) propényl]-β-alanine, 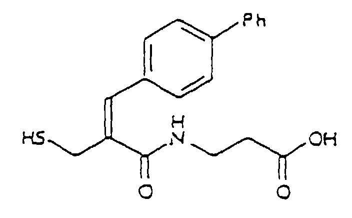
26) le N-(Z)-[1-oxo 2-(acétylthiométhyl) 3-(4-éthoxy phényl) propényl]-β-alaninate de benzyle, 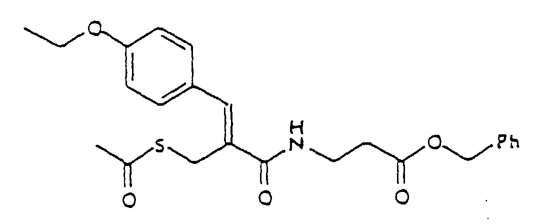
27) le N-(E)-[1-oxo 2-(acétylthiométhyl) 3-(4-éthoxy phényl) propényl]-β-alaninate de benzyle, 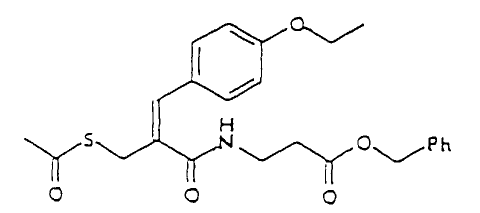
28) la N-(E)-[1-oxo 2-(mercaptométhyl) 3-(4-éthoxy phényl) propényl]-β-alanine, 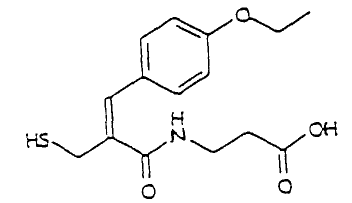
29) le N-(Z)-[1-oxo 2-(acétylthiométhyl) 3-(4-propoxy phényl) propényl]-β-alaninate de benzyle, 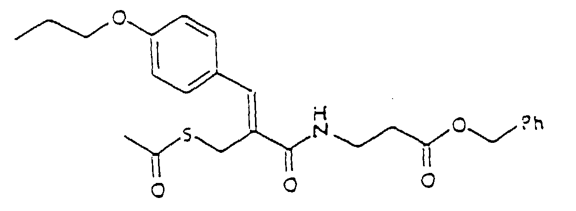
30) le N-(E)-[1-oxo 2-(acétylthiométhyl) 3-(4-propoxy phényl) propényl]-β-alaninate de benzyle, 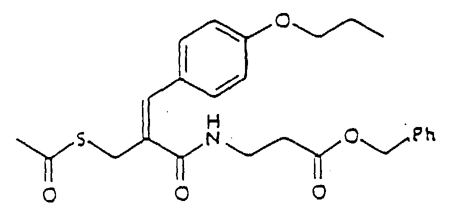
31) le N-(Z)-[1-oxo 2-(acétylthiométhyl) 3-(3,5-difluoro phényl) propényl]-β-alaninate de benzyle, 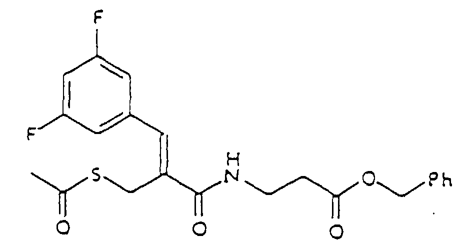
32) le N-(E)-[1-oxo 2-(acétylthiométhyl) 3-(3,5-difluoro phényl) propényl]-β-alaninate de benzyle, 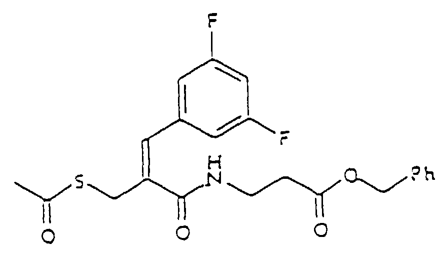
33) la N-(E)-[1-oxo 2-(mercaptométhyl) 3-(3,5-difluoro phényl) propényl]-β-alanine, 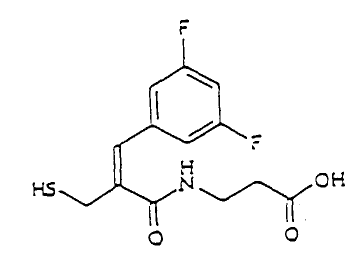
34) le N-(Z)-[1-oxo 2-(acétylthiométhyl) 3-(1-naphtyl) propényl]-β-alaninate de benzyle, 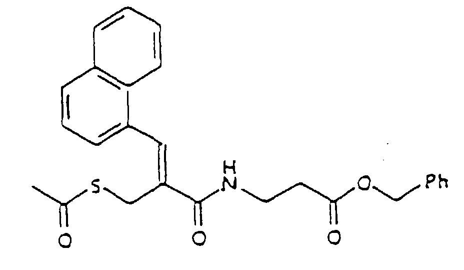
35) le N-(E)-[1-oxo 2-(acétylthiométhyl) 3-(1-naphtyl) propényl]-β-alaninate de benzyle, 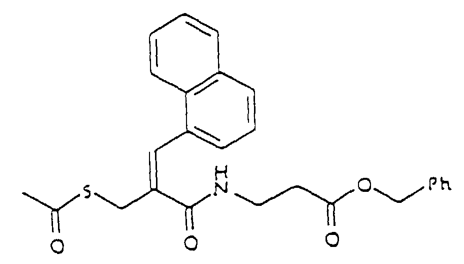
36) la N-(E)-[1-oxo 2-(mercaptométhyl) 3-(1-napthtyl) propényl]-β-alanine, 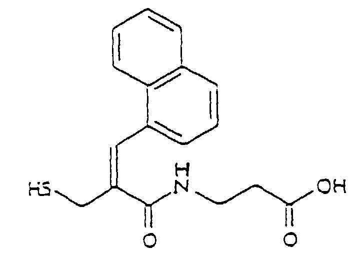
37) le N-(Z)-[1-oxo 2-(acétylthiométhyl) 3-phényl propényl]-β-alaninate de benzyle, 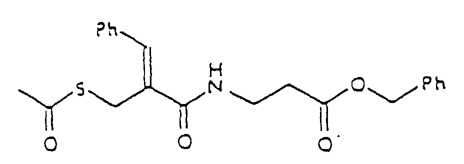
38) la N-(Z)-[1-oxo 2-(mercaptométhyl) 3-phényl propényl]-β-alanine, 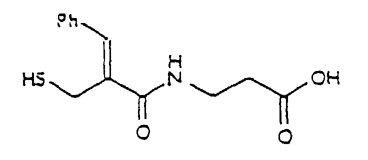
39) le N-(Z)-[1-oxo 2-(acétylthiométhyl) 3-(4-phénoxy phényl) propényl]-β-alaninate de benzyle, 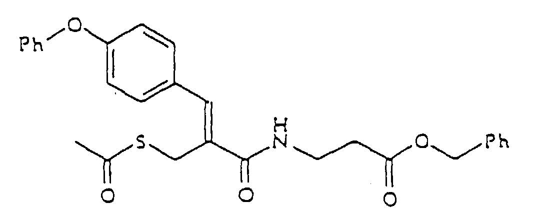
40) le N-(E)-[1-oxo 2-(acétylthiométhyl) 3-(4-phénoxy phényl) propényl]-β-alaninate de benzyle, 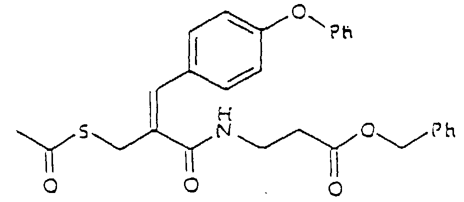
41) la N-(E)-[1-oxo 2-(mercaptométhyl) 3-(4-phénoxy phényl) propényl]-β-alanine, 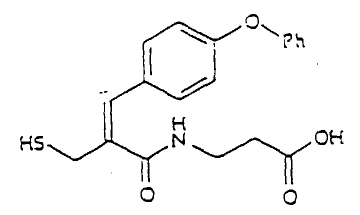
42) le N-(Z)-[1-oxo 2-(acétylthiométhyl) 3-(3,4-méthylènedioxy phényl) propényl]-β-alaninate de benzyle, 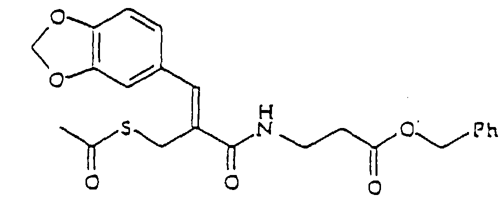
43) le N-(E)-[1-oxo 2-(acétylthiométhyl) 3-(3,4-méthylènedioxy phényl) propényl]-β-alaninate de benzyle,

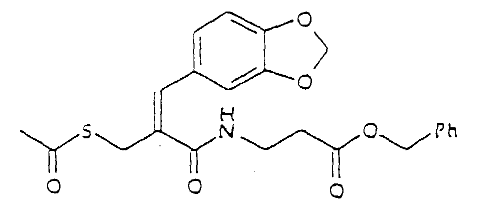

Les composés de formules (Ia) et (Ib) possèdent zéro, un ou deux centres d'asymétrie. Ils existent donc sous forme de mélange racémique ou sous forme optiquement pure. Tous ces composés entrent également dans le cadre de la présente invention.

La présente invention concerne également les procédés de préparation des composés de formules (Ia) et (Ib).

Le procédé de préparation des composés de formule (Ib) (composés qui possèdent une double liaison de configuration (Z)), est caractérisé en ce qu'il consiste successivement :
a) à effectuer une bromation allylique d'un acide éthylénique de configuration (E) de formule (II) 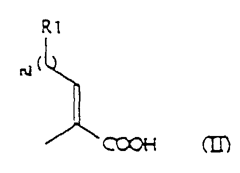 dans laquelle R₁ a la signification précitée, avec un agent de bromation tel que le N-bromosuccinimide en présence d'une quantité catalytique de peroxyde de benzoyle pour former un acide de formule (III) 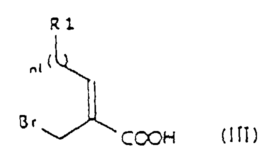
b) à substituer le brome de l'acide de formule (III), de préférence par un thioacide R₄-SH, pour former l'acide éthylénique thioacylé de configuration (Z) de formule (IV) 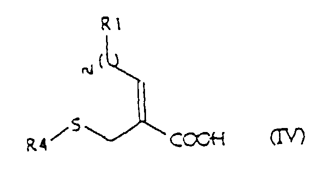 où R₄ a la définition précitée,
c) à coupler l'acide de formule (IV), avec le sel d'amino-ester désiré de formule (V) 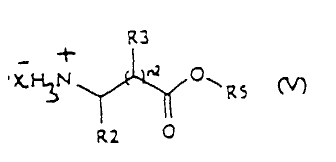 où R₂, R₃, R₅ et n₂ ont les significations précitées et X représente par exemple les halogénures (en particulier le chlorure), le benzènesulfonate, le méthanesulfonate et le toluènesulfonate, pour former le composé de configuration (Z) de formule (Ib) 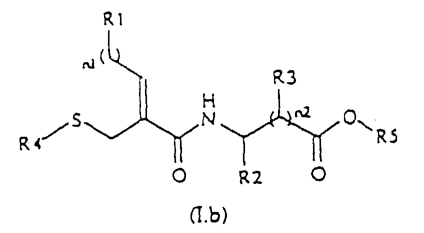 où R₄ et R₅ ne sont pas un atome d'hydrogène,
d) à soumettre le composé de formule (Ib) à une déprotection alcaline pour former les composés de configuration (Z) de formule (Ib) où R₄ et R₅ sont des atomes d'hydrogène

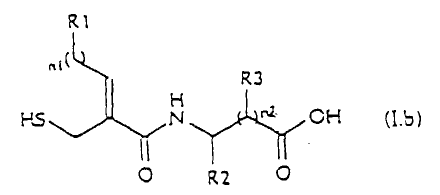

L'acide éthylénique de configuration (E) de formule (II) (étape a) peut être préparé, par exemple, par une réaction de Perkin telle que décrite dans Org. React. 1, 210-216, 1942.

La substitution du brome de l'acide de formule (III) par un thioacide (étape b) peut s'effectuer selon une méthode utilisant du carbonate de potassium et de l'hydrogénocarbonate de sodium dans l'eau ou selon une méthode utilisant la diisopropyléthylamine dans le tétrahydrofuranne.

La réaction de condensation sous c) (couplage de l'acide de formule (IV) avec le sel d'amino-ester de formule (V)) s'effectue en utilisant, de préférence, le procédé au dicyclohexylcarbodiimide/1-hydroxy-benzotriazole (DCC/HOBT).

La déprotection alcaline sous d) s'effectue, de préférence, avec une solution aqueuse d'hydroxyde de sodium dans l'alcool méthylique.

Le procédé de préparation des composés de formule (Ia) (double liaison de configuration (E)) est caractérisé en ce qu'il consiste successivement :
a) à isomériser l'acide éthylénique de configuration (Z) de formule (IV) 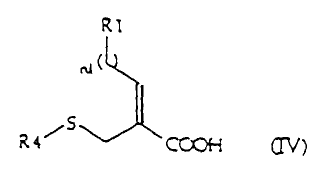 où R₄, R₁ et n₁ ont les significations précitées, à l'aide par exemple d'une lampe à ultra-violet (U.V.), puis à séparer le mélange d'isomères (E/Z) obtenu, par une amine telle que la cyclohexylamine, pour obtenir l'acide éthylénique thioacylé de configuration (E) de formule (VI) 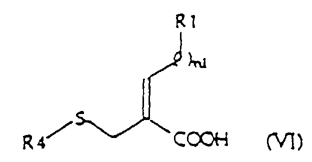
b) à coupler l'acide de formule (VI) avec le sel d'amino-ester désiré de formule (V), en présence d'un agent de couplage tel que le dicyclohexylcarbodiimide, pour obtenir le composé de formule (Ia) de configuration (E) où R₄ et R₅ ne sont pas des atomes d'hydrogène 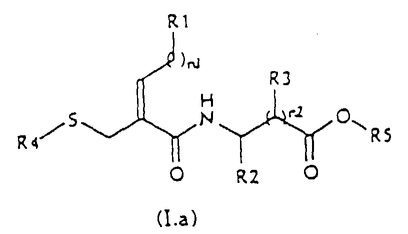
c) puis à soumettre le composé de formule (Ia) à une déprotection alcaline, pour obtenir les composés de configuration (E) de formule (Ia) où R₄ et R₅ sont des atomes d'hydrogène

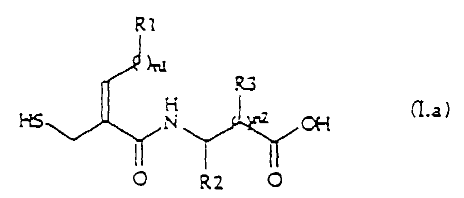

La déprotection alcaline à laquelle est soumise le composé de formule (Ia) s'effectue, de préférence, en utilisant la lithine dans des solvants mixtes tels que le tétrahydrofuranne et l'eau.

Selon une variante de réalisation, les composés de formule (Ia) sont préparés :
a) en isomérisant l'acide éthylénique de formule (IV) possédant une configuration (Z) à l'aide d'une lampe à ultra-violet (U.V.) pour obtenir l'acide thioacylé éthylénique sous forme d'un mélange d'isomères de configuration (Z/E) de formule (VII) 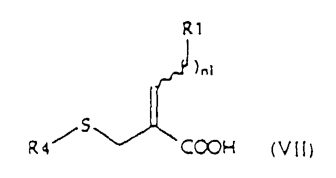 où R₄, R₁ et n₁ ont les significations précitées,
b) en couplant l'acide de formule (VII) avec le sel d'amino-ester désiré de formule (V) en présence d'un agent de couplage tel que le dicyclohexylcarbodiimide pour obtenir le composé de formule (VIII) constitué d'un mélange d'isomères (Z/E) 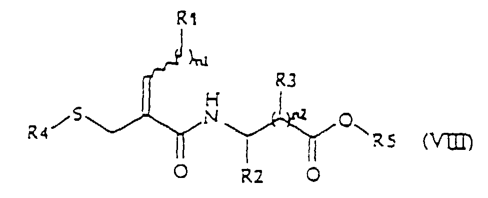
c) en séparant le mélange d'isomères (Z/E) du composé de formule (VIII), par exemple par une chromatographie éclair sur silice ou par recristallisation fractionnée pour obtenir le composé de configuration (E) de formule (Ia) où R₄ et R₅ ne sont pas des atomes d'hydrogène

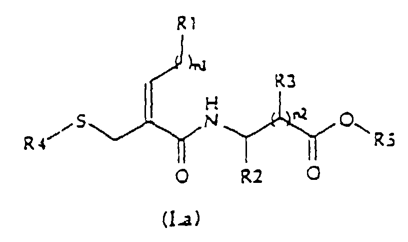

Selon une autre variante de réalisation, les composés de formule (Ia) où R₄ et R₅ ne sont pas l'hydrogène, sont préparés ;
a) en isomérisant le composé de formule (Ib) possédant une double liaison de configuration (Z) 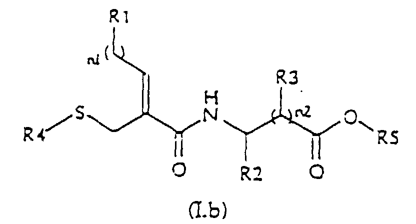 à l'aide par exemple d'une lampe à ultra-violet (U.V.), de préférence en présence d'éthérate de trifluorure de bore, pour obtenir le composé de formule (VIII) constitué d'un mélange d'isomères (Z/E) 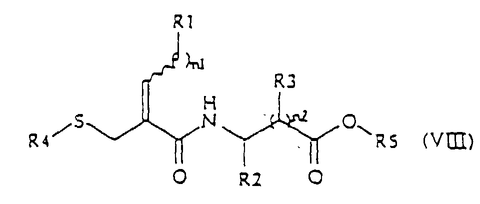
b) en séparant le mélange d'isomères (Z/E) du composé de formule (VIII), par exemple par une chromatographie éclair sur silice ou par recristallisation fractionnée, pour obtenir le composé de configuration (E) de formule (Ia) où R₄ et R₅ ne sont pas des atomes d'hydrogène

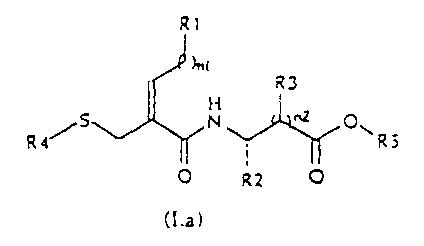

Il va être donné, ci-dessous, à titre non limitatif, plusieurs exemples de mise en oeuvre de l'invention. Dans les exemples donnés ci-dessous, on a utilisé pour la description des spectres les abréviations suivantes :
s : singulet t : triplet m : multiplet
d : doublet q : quadruplet app : apparent.

Les déplacements chimiques sont exprimés en ppm. Les points de fusion sont mesurés sur un banc KOFLER.

### Exemple 1 : Préparation du N-(E)-[1-oxo 2-(acétylthiométhyl) 3-phényl propényl]-β-alaninate de benzyle

A) Préparation de l'acide (Z) 2-bromométhyl 3-phényl propénoïque
   On chauffe à reflux pendant 6 heures un mélange de 2 g (12,34 mmol) d'acide (E)2-méthyl cinnamique, 2,19 g (12,34 mmol) de N-bromosuccinimide (NBS) et une quantité catalytique de peroxyde de benzoyle dans 6 ml de CCl₄. On filtre et on lave le résidu à l'éther. On lave la phase organique successivement par une solution aqueuse d'HCl 1N, puis par de l'eau. On sèche sur MgSO₄, on filtre et on évapore à sec.
   Rendement = 57 % (recristallisé dans l'éther)
   F : 168°C
   IR (nujol) : 1665 cm⁻¹
   RMN¹H (CDCl₃/TMS) : 10,0 (s, 1H) ; 7,9 (s, 1H) ; 7,8 à 7,2 (m, 5H) ; 4,0 (s, 2H).
B) Préparation de l'acide (Z)2-acétylthiométhyl 3-phényl propénoïque.
   On mélange 2,1 g de l'acide obtenu précédemment (8,70 mmol), 0,73 g (8,70 mmol) de NaHCO₃ et 3 ml d'eau. On ajoute à 0°C, une solution de 0,67 g (8,8 mmol) d'acide thioacétique et de 1,44 g (10,43 mmol) de K₂CO₃ dans 21 ml d'eau. On agite pendant 15 heures à 20°C. On acidifie par une solution aqueuse d'HCl 6N. On extrait deux fois à l'éther. Les phases éthérées réunies sont lavées à l'eau, séchées sur MgSO₄, filtrées et concentrées.
   Rendement = 67 % (recristallisé dans l'éther)
   F = 114°C
   IR (nujol) : 1670 cm⁻¹
   RMN¹H (CDCl₃/TMS) : 9,55 (s, 1H) ; 8,00 (s, 1H) ; 7,50 (s,5H); 4,10 (s,2H) ; 2,30 (s,3H).
C) Préparation de l'acide (E) 2-acétylthiométhyl 3-phényl propénoïque
   On irradie pendant 16 heures, avec une lampe TQ 150 Hanovia, 2g de l'acide (Z) précédent en solution dans 30 ml d'éthanol. Après évaporation, on obtient un mélange d'acide Z/E dans un rapport 6/4. Ce mélange est repris par 25 ml d'éther et on ajoute une solution de 0,39 g de cyclohexylamine (0,4 éq.) dans 5 ml d'éther. Après 30 minutes sous agitation, on filtre. Le sel récupéré est traité par une solution aqueuse d'HCl 3N. On extrait à l'éther. La phase organique est lavée par une solution aqueuse saturée de NaCl, séchée sur MgSO₄, filtrée et concentrée.
   Rendement = 32 %
   F = 57°C
   RMN¹H (CDCl₃/TMS) : 9,60 (s, 1H) ; 7,30 (s, 5H) ; 7,20 (s,1H) ; 3,85 (s, 2H) ; 2,25 (s, 3H).
D) Préparation du N-(E)-[1-oxo 2-(acétylthiométhyl) 3-phényl propényl]-β-alaninate de benzyle.
   Dans un ballon muni d'une garde à chlorure de calcium, on place 0,95 g (4 mmol) d'acide (E)2-acétylthiométhyl 3-phényl propénoïque en solution dans 15 ml de THF anhydre. On refroidit le ballon à environ 0-5°C par un bain de glace et on ajoute successivement sous agitation 1,10 g (4 mmol) de méthanesulfonate de β-alaninate de benzyle et 0,41 g (4 mmol) de triéthylamine dans 5 ml de chloroforme, une solution de 0,54 g (4 mmol) d'hydroxy-benzotriazole monohydraté dans 5 ml de THF et une solution de 0,83 g (4 mmol) de dicyclohexylcarbodiimide dans 5 ml de chloroforme. On laisse le mélange revenir à température ambiante et on agite pendant 6 heures.

Le précipité de dicyclohexylurée (DCU) est filtré et on évapore à sec. Le résidu pâteux est repris par l'acétate d'éthyle (12 ml). La DCU qui a à nouveau précipité est filtrée. La phase organique est successivement lavée à l'eau (1 fois 10 ml), par une solution aqueuse saturée d'hydrogénocarbonate de sodium (3 fois 10 ml), à l'eau (1 fois 10 ml) et par une solution aqueuse saturée de NaCl (1 fois 10 ml). On sèche sur MgSO₄, on filtre et on concentre. On obtient un résidu solide blanc que l'on dissout dans le minimum d'éther. Après recristallisation, on obtient 1,25 g d'un solide blanc.
Rendement : 78 % (recristallisé dans l'éther)
F = 62°C
IR (nujol) : 3220, 1730, 1670, 1650, 1620 cm⁻¹
RMN¹H(CDCl₃) : 7,35 à 7,15 (m, 10 H) ; 6,80 (s, 1H) ; 5,95 (t, 1H, J = 5,1 Hz) ; 4,95 (s, 2H) ; 3,85 (d, 2H, J = 1 Hz); 3,45 (q app, 2H, J = 6,10 Hz) ; 2,45 (t, 2H, J = 6,0 Hz) ; 2,30 (s, 3H)
RMN¹³C (CDCl₃) : 194,8 ; 171,7 ; 168,2 ; 135,4 ; 134,9 ; 134,2 ; 132,1 ; 128,5 ; 128,3 ; 128,1 66,3 ; 34,5 ; 33,4 ; 33,1 ; 30,5.

| Microanalyse : C₂₂H₂₃O₄NS | | | |
|---|---|---|---|
| Calc. % | C = 66,48 | H = 5,83 | N = 3,52 |
| Tr. % | C = 66,34 | H = 5,82 | N = 3,53 |

### Exemple 2 : Préparation du N-(E)-[1-oxo 2-(acétylthiométhyl) 3-phényl propényl] - β-alaninate de méthyle.

On couple l'acide (E) 2-acétylthiométhyl 3-phényl propénoïque décrit dans l'exemple 1 (étape C) avec le β-alaninate de méthyle, selon le mode opératoire décrit à l'exemple 1 (étape D).
Rendement : 60 % (après chromatographie éclair, éluant : éther-éther de pétrole 6/4)
F = 54°C
IR (nujol) : 3280, 1720, 1680, 1630, 1610 cm⁻¹
RMN¹H : 7,20 (s,5H) ; 6,80 (s,1H) ; 5,90 (s large, 1 H) ; 3,85 (s, 2H) ; 3,50 (s, 3H) ; 3,60 à 3,30 (m, 2H) ; 2,35 (t, 2H, J = 5,30 Hz) ; 2,25 (s, 3H).

| Microanalyse : C₁₆H₁₉O₄NS | | | |
|---|---|---|---|
| Calc. % | C = 59,79 | H = 5,96 | N = 4,36 |
| Tr. % | C = 59,67 | H = 6,28 | N = 4,47 |

### Exemple 3 : Préparation du N-(E)-[1-oxo 2-(acétylthiométhyl) 3-phényl propényl]-β-alaninate d'éthyle

On couple l'acide (E) 2-acétylthiométhyl 3-phényl propénoïque décrit dans l'exemple 1 (étape C) avec le β-alaninate d'éthyle selon le mode opératoire décrit à l'exemple 1 (étape D)
Rendement = 68 % (chromatographié sur silice, éluant : éther-éther de pétrole 6/4)
F < 50°C.
IR (nujol) : 3300, 1730, 1690, 1640, 1610 cm⁻¹
RMN¹H (CDCl₃/TMS) = 7,20 (s, 5H) ; 6,75 (s, 1H) ; 6,00 (s large, 1H) ; 3,95 (Q, 2H, J=6,7 Hz) ; 3,85 (s, 2H) ; 3,40 (Q app., 2H, J app. = 6,2 Hz) ; 2,30 (t, 2H, J = 6,7 Hz) ; 2,30 (s, 3H) ; 1,15 (t, 3H, J = 6,7 Hz).

| Microanalyse : C₁₇ H₂₁ O₄ NS | | | |
|---|---|---|---|
| Calc. % | C = 60,87 | H = 6,31 | N = 4,18 |
| Tr. % | C = 60,12 | H = 6,22 | N = 3,92 |

### Exemple 4 : Préparation de la N-(E)-[1-oxo 2-(mercaptométhyl) 3-phényl propényl]-β-alanine

A une solution de 2 mmol du diester obtenu à l'exemple 1 (étape D) en solution dans un mélange THF - H₂O (75-25), on ajoute à 0°C, sous atmosphère d'argon, 8 mmol de LiOH et on agite 2 heures. On évapore le THF, on lave la phase aqueuse à l'éther, et on acidifie avec une solution aqueuse d'HCl 3N. On extrait à l'éther, on lave avec une solution aqueuse saturée de NaCl, on sèche sur MgSO₄. On filtre et on évapore.
Rendement : 63 % (après chromatographie éclair, éluant : éther)
F = 80°C
IR (nujol) : 3340, 1700, 1630, 1610 cm⁻¹.
RMN¹H (acétone D6) : 10,90 à 10,60 (m,1H) ; 7,65 (m, 1H) ; 7,45 à 7,35 (m,5H) ; 7,20 (s, 1H) ; 3,75 à 3,50 (m, 4H) ; 2,60 (t, 2H, J = 6,75 Hz) ; 2,30 (t, 1H, J = 7,50 Hz)

| Microanalyse : C₁₃H₁₅O₃NS | | | |
|---|---|---|---|
| Calc. % | C = 58,85 | H = 5,70 | N = 5,28 |
| Tr. % | C = 58,68 | H = 5,78 | N = 5,17 |

### Exemple 5 : Préparation du N-(E)-[1-oxo-2-(benzoylthiométhyl) 3-phényl propényl] - β-alaninate de benzyle

A) Préparation de l'acide (Z)2-benzoylthiométhyl 3-phényl propénoïque.
On fait réagir l'acide (Z) 2-bromométhyl 3-phényl propénoïque décrit dans l'exemple 1 (étape A) avec de l'acide thiobenzoïque selon le mode opératoire décrit à l'exemple 1 (étape B).
Rendement = 67 % (recristallisé dans l'éther)
F = 160°C
RMN¹H (CDCl₃/TMS) : 8,15 à 7,80 (m, 3H) ; 7,70 à 7,20 (m, 8H) ; 4,20 (s, 2H)

B) Préparation du mélange (Z/E) de l'acide 2-benzoylthiométhyl 3-phényl propénoïque.
On irradie pendant 16 heures, avec une lampe TQ 150 Hanovia, 2g de l'acide (Z) précédent en solution dans 20 ml d'éthanol. Après évaporation, on obtient un mélange d'acide Z/E (60/40).
RMN¹H (CDCl₃/TMS) : 9,00 (s,1H) ; 8,20 à 6,8 (m, 11 H) ; 4,25 (s, 1,2H); 4,10 (s, 0,8 H).

C) Préparation du N-(E)-[1-oxo 2-(benzoylthiométhyl) 3-phényl propényl] - β - alaninate de benzyle.
On couple l'acide (Z/E) 2-benzoylthiométhyl 3-phényl propénoïque décrit dans l'exemple 5 (étape B) avec le β-alaninate de benzyle selon le mode opératoire décrit à l'exemple 1 (étape D). L'isomère (E) est ensuite purifié par chromatographie éclair avec le mélange éther-éther de pétrole (50/50) comme éluant.
Rendement : 16 %
F = 60°C
IR (nujol) : 3320, 1720, 1650, 1620 cm⁻¹
RMN¹H (CDCl₃/TMS) = 8,05 à 7,80 (m , 2 H) ; 7,60 à 7,00 (m, H) ; 6,90 (s, 1H) ; 6,00 (t, large, 1H, J = 6,2 Hz) ; 4,90 (s, 2H) ; 4,05 (s, 2H) ; 3,4 (Q app, 2H, J app. = 6,7 Hz) ; 2,40 (t, 2H, J = 5,3 Hz).

| Microanalyse : C₂₇H₂₅O₄NS | | | |
|---|---|---|---|
| Calc. % | C = 70,57 | H = 5,48 | N = 3,05 |
| Tr. % | C = 70,18 | H = 5,63 | N = 2,86 |

### Exemple 6 : Préparation du N-(E)-[1-oxo 2-(benzoylthiométhyl) 3-phényl propényl]-β-alaninate de méthyle.

On couple l'acide (Z/E) 2-benzoylthiométhyl 3-phényl propénoïque décrit dans l'exemple 5 (étape B) avec le β-alaninate de méthyle selon le mode opératoire décrit à l'exemple 1 (étape D). L'isomère (E) est ensuite purifié par chromatographie éclair avec le mélange éther-éther de pétrole (60/40) comme éluant.
Rendement = 16 %
F = 66°C
IR (nujol) : 3280, 1730, 1640, 1620 cm⁻¹
RMN¹H (CDCl₃) : 8,10 à 7,80 (m, 2H) ; 7,70 à 7,10 (m, 8H) ; 6,90 (s, 1H) ; 6,00 (t large, 1H) ; 4,05 (s, 2H) ; 3,70 à 3,10 (m, 2H) ; 3,40 (s, 3H) ; 2,35 (t, 2H , J = 5,4 Hz).

| Microanalyse :C₂₁H₂₁O₄NS | | | |
|---|---|---|---|
| Calc. % | C = 65,78 | H = 5,52 | N = 3,65 |
| Tr. % | C = 65,51 | H = 5,75 | N = 3,20 |

### Exemple 7 : Préparation du N-(E)-[1-oxo 2-(benzoylthiométhyl) 3-phényl propényl ]-β-alaninate d'éthyle.

On couple l'acide (Z/E)2-benzoylthiométhyl 3-phényl propénoïque décrit dans l'exemple 5 (étape B) avec le β-alaninate d'éthyle selon le mode opératoire décrit à l'exemple 1 (étape D). L'isomère (E) est ensuite purifié par chromatographie éclair avec le mélange éther-éther de pétrole (60/40) comme éluant.
Rendement = 13 %
F < 50°C
RMN¹H(CDCl₃) : 8,10 à 7,80 (m, 2 H) ; 7,70 à 7,10 (m, 8H) ; 6,90 (s, 1H) ; 6,00 (t large, 1 H) ; 4,05 (s, 2H) ; 3,90 (Q, 2H, J = 7,5 Hz) ; 3,45 (Q app, 2H, J app = 5,3 Hz) ; 2,35 (t, 2H, J = 5,3 Hz) ; 1,10 (t, 3H, J = 7,5 Hz)

| Microanalyse C₂₂ H₂₃O₄NS | | | |
|---|---|---|---|
| Calc. % | C = 66,48 | H = 5,83 | N = 3,52 |
| Tr. % | C = 66,80 | H = 5,92 | N = 3,75. |

### Exemple 8 : Préparation du N - (E) - [1-oxo 2-(pivaloylthiométhyl) 3-phényl propényl]-β- alaninate de benzyle

A) Préparation de l'acide (Z) 2-pivaloylthiométhyl 3 -phényl propénoïque.
On fait réagir l'acide (Z)-2-bromométhyl 3-phényl propénoïque décrit dans l'exemple 1 (étape A) avec de l'acide thiopivaloïque selon le mode opératoire décrit à l'exemple 1 (étape B).
Rendement : 93 %
RMN¹H (CDCl₃) : 9,10 (s large, 1H) ; 7,90 (s, 1H) ; 7,35 (s, 5H) ; 4,00 (s, 2H) ; 1,20 (s, 9H)

B) Préparation de l'acide (E) 2-pivaloylthiométhyl 3-phényl propénoïque
On irradie l'acide (Z) 2-pivaloylthiométhyl 3-phényl propénoïque précédent selon le mode opératoire décrit à l'exemple 1 (étape C). La purification de l'isomère (E) s'effectue selon le mode opératoire décrit à l'exemple 1 (étape C).
Rendement : 24 % (par rapport à l'acide (Z) de départ)
F = 88°C
RNM¹H (CDCl₃) : 8,70 (s large, 1H) ; 7,45 à 7,10 (m, 6H) ; 3,85 (s,2H) ; 1,20 (s, 9H).

C) Préparation du N-(E)-[1-oxo 2-(pivaloylthiométhyl) 3-phényl propényl]-β-alaninate de benzyle
On couple l'acide (E) 2-pivaloylthiométhyl 3-phényl propénoïque précédent avec le β-alaninate de benzyle selon le mode opératoire décrit à l'exemple 1 (étape D).
Rendement : 63 % (après chromatographie éclair, éluant : éther-éther de pétrole 55/45)
huile
IR : 3320, 1725, 1650, 1620 cm⁻¹
RMN¹H (CDCl₃) : 7,30 (s, 5H) ; 7,20 (s, 5H) ; 6,75 (s,1H); 4,90 (s, 2H) ; 3,80 (s, 2H) ; 3,45 (q app ; 2H, J = 6,0 Hz); 2,40 (t, 2H, J = 6,5 Hz) ; 1,20 (s, 9H).

| Microanalyse : C₂₅H₂₉NO₄S | | | |
|---|---|---|---|
| Calc % : | C = 68,31 | H = 6,65 | N = 3,19 |
| Tr. % | C = 68,02 | H = 6,73 | N = 3,01 |

### Exemple 9 : Préparation du N-(E)[1-oxo 2-(pivaloylthiométhyl) 3-phényl propényl]-β-alaninate de méthyle.

On couple l'acide (E) 2-pivaloylthiométhyl 3-phényl propénoïque décrit à l'exemple 8 (étape B) avec le β-alaninate de méthyle selon le mode opératoire décrit à l'exemple 1 (étape D).
Rendement : 69 % (après chromatographie éclair, éluant : éther-éther de pétrole 55/45)
huile
IR : 3300, 1730, 1660, 1630 cm⁻¹
RMN¹H (CDCl₃) : 7,20 (s,5H) ; 6,75 (s,1H) ; 5,90 (t large, 1H) ; 3,80 (s, 2H) ; 3,50 (s, 3H) ; 3,60 à 3,25 (m, 2H) ; 2,40 (t, 2H, J = 6,5 Hz) ; 1,20 (s, 9H)

| Microanalyse : C₁₉H₂₅NO₄S | | | |
|---|---|---|---|
| Calc % : | C = 62,79 | H = 6,93 | N = 3,85 |
| Tr. % : | C = 63,20 | H = 7,10 | N = 3,92 |

### Exemple 10 : Préparation du N-(E)-[1-oxo 2-(pivaloylthiométhyl) 3-phényl propényl]-β-alaninate d'éthyle

On couple l'acide (E) 2-pivaloylthiométhyl 3-phényl propénoïque décrit à l'exemple 8 (étape B) avec le β-alaninate d'éthyle selon le mode opératoire décrit à l'exemple 1 (étape D)
Rendement 73 % (après chromatographie éclair, éluant : éther-éther de pétrole 55/45) - huile
IR : 3300, 1710, 1650, 1620 cm⁻¹
RMN¹H (CDCl₃) : 7,20 (s, 5H) ; 6,75 (s, 1H) ; 6,0 (t large, 1H) ; 3,95 (q, 2H, J = 7,5 Hz) ; 3,80 (s, 2H) ; 3,40 (q app., 2H, J. app. = 6,40 Hz) ; 2,35 (t, 2H, J = 5,9 Hz) ; 1,30 à 0,95 (m, 12 H).

| Microanalyse : C₂₀ H₂₇ NO₄S | | | |
|---|---|---|---|
| Calc % : | C = 63,63 | H = 7,21 | N = 3,71 |
| Tr. % | C = 63,35 | H = 7,16 | N = 3,52 |

### Exemple 11 : Préparation du N-(E)-[1-oxo 2-(acétylthiométhyl) 3-phényl propényl]-(RS)-3-amino butanoate de méthyle.

On couple l'acide (E) 2-acétylthiométhyl 3-phényl propénoïque décrit à l'exemple 1 (étape C) avec le (RS)-3-amino butanoate de méthyle selon le mode opératoire décrit à l'exemple 1 (étape D).
Rendement : 70 % (recristallisé)
F : 98°C
IR (nujol) : 3300, 1730, 1700, 1620 cm⁻¹
RMN¹H (CDCl₃) : 7,20 (s, 5H) ; 6,80 (s, 1 H) ; 5,90 (d large, 1H) ; 4,50 à 4,10 (m, 1H); 3,85 (s, 2H) ; 3,50 (s, 3H) ; 2,30 (s, 5H) ; 1,00 (d, 3H, J = 6,4 Hz).

| Microanalyse : C₁₇ H₂₁ NO₄S | | | |
|---|---|---|---|
| Calc. % | C = 60,87 | H = 6,31 | N = 4,18 |
| Tr % | C = 60,34 | H = 6,27 | N = 3,98 |

### Exemple 12 : Préparation de l'acide N-(E)-[1-oxo 2-(mercaptométhyl) 3-phényl propényl]-(RS)-3-amino butanoïque

On effectue la saponification du diester précédent selon le mode opératoire décrit à l'exemple 4.
Rendement : 63 % (huile)
IR : 3280, 1700, 1640, 1600 cm⁻¹
RMN¹H (CDCl₃) : 10,0 (s, 1H) ; 7,20 (s, 5H) ; 6,65 (s, 1H) ; 6,15 (d, 1H, J = 9,8 Hz) ; 4,80 à 4,10 (m, 1H) ; 3,5 (d, 2H, J = 8,4 Hz); 2,40 (d, 2H, J = 5,6 Hz) : 1,75 (t, 1H, J = 8,4Hz) ; 1,00 (d, 3H, J = 7,0 Hz).

| Microanalyse : C₁₄H₁₇NO₃S | | | |
|---|---|---|---|
| Calc. % | C = 60,19 | H = 6,13 | N = 5,01 |
| Tr. % | C = 59,39 | H = 6,56 | N = 4,71 |

### Exemple 13 : Préparation du N-(E)-[1-oxo 2-(acétylthiométhyl) 3-phényl propényl]-(RS)-3-amino 2-méthyl propanoate de méthyle

On couple l'acide (E) 2-acétylthiométhyl 3-phényl propénoïque décrit à l'exemple 1 (étape C) avec le (RS)-3-amino 2-méthyl propanoate de méthyle selon le mode opératoire décrit à l'exemple 1 (étape D)
Rendement : 60 % (après chromatographie éclair, éluant : éther-éther de pétrole 6/4)
RMN¹H (CDCl₃) : 7,40 à 7,15 (m, 5H) ; 6,75 (s, 1H) ; 5,90 (t large, 1H) ; 3,85 (s, 2H) ; 3,55 à 3,35 (m, 1H) ; 3,50 (s, 3H) ; 3,25 à 3,05 (m, 1H) ; 2,65 à 2,45 (m, 1H) ; 2,35 (s, 3H) ; 1,05 (d, 3H, J = 7,5 Hz)

| Microanalyse : C₁₇H₂₁NO₄S | | | |
|---|---|---|---|
| Calc. % | C = 60,87 | H = 6,31 | N = 4,18 |
| Tr. % | C = 60,53 | H = 6,28 | N = 4,30 |

### Exemple 14 : Préparation de l'acide N-(E)-[1-oxo 2-(mercaptométhyl) 3-phényl propényl]-(RS)-3-amino 2-méthyl propanoïque

On effectue la saponification du diester décrit à l'exemple 13 selon le mode opératoire décrit à l'exemple 4.
Rendement : 60 % (après chromatographie éclair, éluant : éther) - huile
RMN¹H (CDCl₃) : 7,40 à 7,10 (m, 6H) ; 6,85 (t large, 1H) ; 6,55 (s, 1H) ; 3,65 à 3,30 (m, 4H) ; 2,55 à 2,40 (m, 1H) ; 1,70 (t, 1H,J = 7,5 Hz) ; 1,05 (d, 3H, J = 7,5 Hz)

| Microanalyse : C₁₄H₁₇NO₃S | | | |
|---|---|---|---|
| Calc. % | C = 60,19 | H = 6,13 | N = 5,01 |
| Tr. % | C = 59,48 | H = 5,99 | N = 4,79 |

### Exemple 15 : Préparation du N-(E)-[1-oxo 2-(acétylthiométhyl) 3-phényl propényl]-4-amino butanoate de benzyle

On couple l'acide (E) 2-acétylthiométhyl 3-phényl propénoique décrit à l'exemple 1 (étape C) avec le 4-amino butanoate de benzyle selon le mode opératoire décrit à l'exemple 1 (étape D).
Rendement :62 % (après chromatographie éclair, éluant : éther-éther de pétrole 7/3)
F = 86°C
IR (nujol) : 3300, 1720, 1685, 1640, 1610 cm⁻¹
RMN¹H (CDCl₃) : 7,45 à 7,15 (m, 10 H) ; 6,80 (s, 1H) ; 5,55 (t large, 1H) ; 5,05 (s,2H) ; 3,85 (s, 2H) ; 3,20 (q. app., 2H, J = 6,0 Hz) ; 2,30 (s, 3H) ; 2,20 (t, 2H, J = 7,5 Hz) ; 1,75 à 1,50 (m, 2H)

| Microanalyse : C₂₃H₂₅NO₄S | | | |
|---|---|---|---|
| Calc. % | C = 67,13 | H = 6,12 | N = 3,40 |
| Tr. % | C = 67,07 | H = 6,07 | N = 3,46 |

### Exemple 16 : Préparation de l'acide N-(E)-[1-oxo 2-(mercaptométhyl) 3-phényl propényl]-4-amino butanoïque

On effectue la saponification du diester décrit à l'exemple 15 selon le mode opératoire décrit à l'exemple 4.
Rendement : 58 % (après chromatographie éclair, éluant : éther) - huile
RMN¹H (CDCl₃) : 7,4 à 7,10 (m, 6H) ; 6,65 (s, 1H) ; 5,70 (s large, 1H) ; 3,50 (d, 2H, J = 7,5 Hz) ; 3,25 (q app., 2H, J = 6 Hz) ; 2,20 (t, 2H, J = 7,5 Hz) ; 1,80 à 1,55 (m, 3H)

| Microanalyse : C₁₄H₁₇NO₃S | | | |
|---|---|---|---|
| Calc. % | C = 60,19 | H = 6,13 | N = 5,01 |
| Tr. % | C = 60,60 | H = 6,32 | N = 5,31 |

### Exemple 17 : Préparation du N-(Z)-[1-oxo 2-(acétylthiométhyl) 3-(4-méthoxy phényl) propényl]-β-alaninate de benzyle

A) Préparation de l'acide (E) 2-méthyl 3-(4-méthoxy phényl) propénoïque.
Un mélange de 68 g de p. anisaldéhyde (500 mmol), de 81 g d'anhydride propionique (625 mmol) et de 48 g de propionate de sodium (500 mmol, séché 5h à 120°C), est chauffé sous argon durant 30 h à 140°C. Cette solution, refroidie à 80°C, est versée lentement dans 400 ml d'une solution aqueuse saturée d'hydrogénocarbonate de sodium fortement agitée. Après extraction avec du dichlorométhane (2 fois 300 ml), les phases organiques sont réunies, puis lavées avec une solution saturée d'hydrogénocarbonate de sodium (2 fois 150 ml). Les phases aqueuses sont réunies, puis versées lentement dans un mélange de 300 ml d'une solution d'acide chlorhydrique 12N et de 100 g de glace pilée. Le précipité est recueilli après filtration, séché au dessicateur, trituré dans 2 fois 150 ml d'éther de pétrole, à nouveau filtré et séché au dessicateur. On recueille 55,70 g (290 mmol) d'un solide blanc.
Rendement = 58 %
F = 178-182°C
IR (nujol) : 1665 cm⁻¹
RMN¹H (CDCl₃/TMS) : 9,20 à 8,90 (s large, 1H) ; 7,60 (s, 1H); 7,35 et 6,90 (AB, 4H, J = 8 Hz) ; 3,80 (s,3H) ; 2,1 (s, 3H)
RMN¹³C (CDCl₃) ; 170,40 ; 158,50 ; 137,60 ; 130,90 ; 128,10; 126,30 ; 113,50 ; 54,80.

B) Préparation de l'acide (Z)-2-bromométhyl 3-(4-méthoxy phényl) propénoïque
On chauffe à reflux pendant 6 heures un mélange de 30 g (156 mmol) de l'acide précédent, 27,80 g (156 mmol) de N-bromosuccinimide (NBS) et une quantité catalytique de peroxyde de benzoyle dans 650 ml de CHCl₃ sous irradiation au moyen d'une lampe halogène (500 W). Après refroidissement à température ambiante, la solution est lavée par une solution chlorhydrique 1N (3 fois 200 ml), décantée, puis séchée sur sulfate de magnésium. On filtre, on concentre et on triture par deux fois dans 350 ml d'un mélange éther/éther de pétrole (1/3). Les eaux-mères sont évaporées et on recueille 39,0 g (144 mmol) d'un solide blanc.
Rendement = 92 % (après trituration dans un mélange éther-éther de pétrole 1/3 / 2/3)
F = 173°C
IR (nujol) : 1670 cm⁻¹
RMN¹H(CDCl₃) : 9,6 à 9,3 (s large, 1H) ; 7,8 (s, 1H) ; 7,55 et 6,90 (AB, 4H, J = 8Hz) ; 4,4 (s, 2H) ; 3,8 (s,3H)

C) Préparation de l'acide (Z)-2-(acétylthiométhyl) 3-(4-méthoxy phényl) propénoïque.
A une solution de 8,40 g (31 mmol) de l'acide précédent en solution dans 100 ml de tétrahydrofuranne à 0°C, on ajoute goutte à goutte une solution de 4,0 g (31 mmol) de diisopropyléthylamine et de 2,36 g (31 mmol) d'acide thioacétique dans 30 ml de tétrahydrofuranne. A la fin de l'addition, on filtre la solution et le filtrat est concentré. On reprend avec 80 ml de dichlorométhane et on lave avec 60 ml d'une solution chlorhydrique 1N. Après décantation, la phase organique est séchée sur sulfate de magnésium, filtrée et concentrée. On récupère 8,09 g (30,4 mmol) d'un solide jaune pâle.
Rendement : 98 %
F = 155°C
IR (nujol) : 1690, 1675, 1615 cm⁻¹
RMN¹H (CDCl₃) : 10,50 à 10,00 (s large, 1H) ; 7,85 (s, 1H) ; 7,40 et 6,90 (AB, 4H, J = 8Hz) ; 4,05 (s, 2H) 3,80 (s, 3H); 2,35 (s, 3H).

D) Préparation du N-(Z)-[1-oxo 2-(acétylthiométhyl) 3-(4-méthoxy phényl) propényl]-β-alaninate de benzyle
On couple l'acide (Z)-2-acétylthiométhyl 3-(4-méthoxy phényl)-propénoïque obtenu à l'étape C avec le β-alaninate de benzyle selon le mode opératoire décrit à l'exemple 1 (étape D).
Rendement 74 % (cristallisation dans un mélange dichlorométhane-éther de pétrole)
F = 96°C
IR (nujol) : 3280, 1740, 1690, 1640, 1620 cm⁻¹
RMN¹H (CDCl₃/TMS) : 7,65 (s, 1H) : 7,35 à 7,15 (m, 7H) ; 6,95 à 6,80 (m, 3H) ; 5,10 (s, 2H) ; 4,00 (s, 2H) ; 3,75 (s, 3H); 3,60 (q, 2H, J app. = 6Hz) ; 2,60 (t, 2H, J = 6 Hz) ; 2,30 (s, 3H).
RMN¹³C (CDCl₃) : 196,2 ; 172,0 ; 167,1 ; 159,8 : 137,9 ; 135,6 ; 130,9 ; ; 128,5 ; 128,2 ; 128,1 ; 127,2 ; 114,1 ; 66,3; 55,2 ; 35,6 ; 33,8 ; 30,2 ; ; 26,6.

| Microanalyse : C₂₃H₂₅O₅NS | | | |
|---|---|---|---|
| Calc. % | C = 64,61 | H = 5,89 | N = 3,27 |
| Tr. % | C = 64,20 | H = 5,73 | N = 3,48 |

### Exemple 18 : Préparation du N-(E)-[1-oxo 2-(acétylthiométhyl)3-(4-méthoxy phényl) propényl]-β-alaninate de benzyle

On dissout 4,93 g (11,5 mmol) du diester (Z) obtenu à l'exemple 17 (étape D) dans 100 ml de chlorure de méthylène passé sur alumine basique. On irradie pendant 2,5 h la solution avec une lampe TQ 150 Hanovia après avoir ajouté au goutte à goutte 1,53 ml d'une solution d'éthérate de trifluorure de bore (11,5 mmol). Puis, on lave la solution avec une solution chlorhydrique 1N (2 fois 80 ml), une solution saturée d'hydrogénocarbonate de sodium (2 fois 80 ml) et on sèche sur sulfate de magnésium. Après filtration et évaporation, on récupère après chromatographie éclair 2,56 g d'isomère (E).
Rendement = 52 % (après chromatographie éclair, éluant éther-éther de pétrole 65/35)
F < 50°C
IR (nujol) : 3290, 1740, 1690, 1630 cm⁻¹
RMN¹H (CDCl₃) : 7,35 à 7,05 (m, 7H) ; 6,75 (AB, 2H, J = 8Hz); 6,70 (s, 1H) ; 6,10 (t,1H, J = 5Hz) ; 4,95 (s, 2H) ; 3,80 (s, 3H) ; 3,40 (q app, 2H, J app. = 6Hz) ; 2,45 (t, 2H, J = 6Hz) ; 2,25 (s, 3H).
RMN¹³C (CDCl₃) : 194,8 ; 171,6 ; 168,6 ; 159,4 ; 135,4 ; 132,0 ; 131,6 ; 129,8 ; 128,5 ; 128,2 ; 128,0 ; 127,2 ; 113,6 ; 66,2 ; 55,0 ; 34,6 ; 33,6 ; 33,2 ; 30,4

| Microanalyse : C₂₃H₂₅O₅NS | | | |
|---|---|---|---|
| Calc. % | C = 64,61 | H = 5,89 | N = 3,27 |
| Tr. % | C = 64,17 | H = 5,91 | N = 3,48 |

### Exemple 19 : Préparation de la N-(E)-[1-oxo 2-(mercaptométhyl) 3-(4-méthoxy phényl) propényl]-β-alanine.

On effectue la saponification du diester (E) obtenu à l'exemple 18 selon le mode opératoire décrit à l'exemple 4.
Rendement : 69 % (après chromatographie éclair, éluant : éther)
F = 160°C
IR (nujol) : 3340, 1730, 1620, 1580 cm⁻¹
RMN¹H (CDCl₃) : 9,10 (s large, 1 H) ; 7,75 et 7,15 (AB, 4H, J = 8Hz) ; 6,60 (s, 1H) ; 6,15 (t large, 1H) : 3,75 (s, 3H); 3,50 à 3,45 (m, 4H) ; 2,50 (t, 2H, J = 5 Hz) : 1,70 (t; 1H, J = 7Hz).

| Microanalyse : C₁₄H₁₇O₄NS | | | |
|---|---|---|---|
| Calc. % | C = 56,92 | H = 5,80 | N = 4,74 |
| Tr. % | C = 57,11 | H = 5,86 | N = 4,91 |

### Exemple 20 : Préparation du N-(Z)-[1-oxo 2-(acétylthiométhyl) 3-(4-trifluorométhyl phényl) propényl]-β-alaninate de benzyle.

A) Préparation de l'acide (E) 2-méthyl 3-(4-trifluorométhyl phényl) propénoïque
On procède de façon analogue à celle décrite à l'exemple 17 (étape A) en partant du 4-trifluorométhylbenzaldéhyde
Rendement = 88 %
F = 170°C
RMN¹H (CDCl₃ / TMS) : 10,55 à 10,00 (s large, 1H) ; 7,75 à 7,35 (m, 5H) ; 2,05 (s, 3H)

B) Préparation de l'acide (Z)-2-bromométhyl 3-(4-trifluorométhyl phényl) propénoïque.
On procède de façon analogue à celle décrite à l'exemple 17 (étape B).
Rendement : 91 %
F : 183°C
IR (nujol) : 1665 cm⁻¹
RMN¹H (CDCl₃/TMS) : 8,20 à 8,00 (s large, 1H) ; 7,80 (s, 1H); 7,70 à 7,55 (m, 4H) 4,30 (s, 2H).

C) Préparation de l'acide (Z)-2-(acétylthiométhyl) 3-(4-trifluorométhyl phényl) propénoïque.
On procède de façon analogue à celle décrite à l'exemple 17 (étape C)
Rendement = 82 %
F = 132°C
IR (nujol) : 1690, 1670 cm⁻¹
RMN¹H (CDCl₃/TMS) : 11,20 à 11,00 (s large, 1H) ; 7,90 (s, 1H) ; 7,70 et 7,50 (AB, 4H, J = 10 Hz) ; 4,05 (s, 2H) ; 2,35 (s,3H).

D) Préparation du N-(Z)-[1-oxo 2-(acétylthiométhyl) 3-(4-trifluorométhyl phényl) propényl]-β-alaninate de benzyle.
On couple l'acide (Z)-2-acétylthiométhyl 3-(4-trifluorométhyl phényl) propénoïque obtenu à l'étape C avec le β-alaninate de benzyle selon le mode opératoire décrit à l'exemple 1 (étape D).
Rendement = 47 % (purification par chromatographie éclair, éluant : éther-éther de pétrole 6/4)
F = 79°C
IR(nujol) : 3280, 1740, 1690, 1640, 1620 cm⁻¹
RMN¹H(CDCl₃/TMS) : 7,70 (s, 1H) ; 7,55 (AB, 2H, J = 8Hz) ; 7,45 à 7,20 (m, 7H) ; 6,95 (t, 1H, J = 6Hz) ; 5,15 (s, 2H) ; 3,90 (s, 2H) ; 3,40 (q app., 2H, J app. = 6Hz) ; 2,65 (t, 2H, J = 5 Hz) 2,30 (s, 3H).

| Microanalyse : C₂₃H₂₂O₄NSF₃ | | | |
|---|---|---|---|
| Calc. % | C = 59,35 | H = 4,76 | N = 3,01 |
| Tr. % | C = 59,17 | H = 4,73 | N = 2,87 |

### Exemple 21 : Préparation du N-(E)-[1-oxo-2-(acétylthiométhyl) 3-(4-trifluorométhyl phényl) propényl]-β-alaninate de benzyle

On procède de façon analogue à celle décrite à l'exemple 18, en partant du diester (Z) obtenu à l'exemple 20 (étape D)
Rendement = 53 % (purification par chromatographie éclair, éluant éther-éther de pétrole 57/43)
F = 76°C
IR (nujol) : 3290, 1740, 1690, 1630 cm⁻¹
RMN¹H (CDCl₃) : 7,45 (AB, 2H, J = 8Hz) ; 7,35 à 7,20 (m, 7H); 6,70 (s, 1H) ; 6,25 (t, 1H, J = 5 Hz) ; 3,80 (s, 2H) ; 3,40 (q. app., 2H, J app. = 6 Hz) ; 2,40 (t, 2H, J = 6 Hz) ; 2,30 (s, 3H).

| Microanalyse : C₂₃H₂₂O₄NSF₃ | | | |
|---|---|---|---|
| Calc. % | C = 59,35 | H = 4,76 | N = 3,01 |
| Tr. % | C = 59,01 | H = 4,51 | N = 3,24 |

### Exemple 22 : Préparation de la N-(E)-[1-oxo 2-(mercaptométhyl) 3-(4-trifluorométhyl phényl) propényl]-β-alanine

On effectue la saponification du diester (E) obtenu à l'exemple 21 selon le mode opératoire décrit à l'exemple 4.
Rendement = 22 %
F = 108°C
IR (nujol) : 1730, 1620, 1580 cm⁻¹
RMN¹H (CDCl₃) : 8,50 (s large, 1H) ; 7,50 à 7,25 (m, 4H) ; 6,65 (s, 1H) ; 6,50 (t large, 1H) ; 3,50 à 3,40 (m, 4H) ; 2,60 (t, 2H, J = 6 Hz) ; 1,80 (t, 1H, J = 8 Hz).

| Microanalyse : C₁₄H₁₄O₃ NSF₃ | | | |
|---|---|---|---|
| Calc. % | C = 50,45 | H = 4,23 | N = 4,20 |
| Tr. % | C = 50,11 | H = 3,99 | N = 4,05 |

### Exemple 23 : Préparation du N-(Z)-[1-oxo 2-(acétylthiométhyl) 3-(4-phényl phényl) propényl]-β-alaninate de benzyle

A) Préparation de l'acide (E) 2-méthyl-3-(4-phényl phényl) propénoïque
On procède de façon analogue à celle décrite à l'exemple 17 (étape A), en partant du 4-phényl benzaldéhyde et en utilisant 6 équivalents d'anhydride propionique.
Rendement : 49 %
F : 178°C
IR (nujol) : 1670 cm⁻¹
RMN¹H (CDCl₃/TMS) : 11,50 à 11,20 (s large, 1H) ; 7,70 (s, 1H) ; 7,60 à 7,20 (m, 9H) ; 2,15 (s, 3H).

B) Préparation de l'acide (Z)-2-bromométhyl 3-(4-phényl phényl) propénoïque.
On procède de façon analogue à celle décrite à l'exemple 17 (étape B)
Rendement : 78 %
F : 169°C
RMN¹H (CDCl₃/TMS) : 7,85 (s, 1H) ; 7,75 à 7,20 (m, 9H) ; 7,00 à 6,70 (s large, 1H) ; 4,40 (s, 2H).

C) Préparation de l'acide (Z)-2-(acétylthiométhyl) 3-(4-phényl phényl) propénoïque.
On procède de façon analogue à celle décrite à l'exemple 17 (étape C).
Rendement : 97 %
F = 158°C
IR (nujol) = 1690, 1670 cm⁻¹
RMN¹H (CDCl₃/TMS) : 10,40 à 10,00 (s large, 1H) ; 7,95 (s, 1H) ; 7,75 à 7,20 (m, 9H) 4,15 (s, 2H) ; 2,35 (s, 3H).

D) Préparation du N-(Z)-[1-oxo-2-(acétylthiométhyl)3-(4-phényl phényl) propényl]-β-alaninate de benzyle.
On couple l'acide (Z)-2-acétylthiométhyl 3-(4-phényl phényl) propénoïque obtenu à l'étape C avec le β-alaninate de benzyle selon le mode opératoire décrit à l'exemple 1 (étape D).
Rendement : 56 % (purification par chromatographie éclair, éluant : éther-éther de pétrole 6/4)
F = 79°C
IR (nujol) : 3280, 1740, 1690, 1640, 1620 cm⁻¹
RMN¹H (CDCl₃/TMS) : 7,75 (s, 1H) ; 7,65 à 7,20 (m, 14 H) ; 7,05 (t, 1H, J = 5Hz) ; 5,0 (s, 2H) ; 4,15 (s, 2H) ; 3,65 (q. app., 2H, J app. = 6H7) ; 2,65 (t, 2H, J = 6Hz) ; 2,30 (s, 3H).

| Microanalyse : C₂₈ H₂₇ O₄ NS | | | |
|---|---|---|---|
| Calc. % | C = 71,01 | H = 5,75 | N = 2,96 |
| Tr. % | C = 71,08 | H = 5,60 | N = 2,46 |

### Exemple 24 : Préparation du N-(E)-[1-oxo 2-(acétylthiométhyl) 3-(4-phényl phényl) propényl]-β-alaninate de benzyle.

On procède de façon analogue à celle décrite à l'exemple 18 en partant du diester (Z) obtenu à l'exemple 23 (étape D).
Rendement = 38 % (purification par chromatographie éclair, éluant : éther-éther de pétrole 6/4)
F = 76°C
IR (nujol) : 3280, 1740, 1690, 1640, 1620 cm⁻¹
RMN¹H (CDCl₃/TMS) : 7,60 à 7,10 (m, 14 H) ; 6,85 (s, 1H) ; 6,20 à 6,00 (m, 1H) ; 5,10 (s, 2H) ; 3,75 (s, 2H) ; 3,40 (q. app., 2H, J. app. = 6Hz) ; 2,45 (t, 2H, J = 6Hz) ; 2,30 (s, 3H).

| Microanalyse : C₂₈H₂₇O₄NS | | | |
|---|---|---|---|
| Calc. % | C = 71,01 | H = 5,75 | N = 2,96 |
| Tr. % | C = 71,29 | H = 5,51 | N = 2,75 |

### Exemple 25 : Préparation de la N-(E)-[1-oxo 2-(mercaptométhyl) 3-(4-phényl phényl) propényl]-β-alanine.

On effectue la saponification du diester (E) obtenu à l'exemple 24 selon le mode opératoire décrit à l'exemple 4.
Rendement : 28 %
F = 125°C
IR (nujol) : 1730, 1620, 1580 cm⁻¹
RMN¹H (CDCl₃/TMS) : 9,70 (s large, 1H) ; 7,70 à 6,80 (m, 11 H) ; 3,80 à 3,40 (m, 4H) ; 2,50 (t, 2H, J = 6Hz) ; 1,70 (t, 1H, J = 8Hz).

| Microanalyse : C₁₉H₁₉O₃NS | | | |
|---|---|---|---|
| Calc. % | C = 66,84 | H = 5,61 | N = 4,10 |
| Tr. % | C = 66,45 | H = 5,29 | N = 3,79 |

### Exemple 26 : Préparation du N-(Z)-[1-oxo 2-(acétylthiométhyl) 3-(4-éthoxy phényl) propényl]-β-alaninate de benzyle

A) Préparation de l'acide (E) 2-méthyl 3-(4-éthoxy phényl) propénoïque
On procède de façon analogue à celle décrite à l'exemple 17 (étape A), en partant du 4-éthoxy benzaldéhyde.
Rendement = 54 %
F = 180°C
IR (nujol) : 1670 cm⁻¹
RMN¹H (CDCl₃/TMS) : 7,65 (s, 1H) ; 7,45 et 6,85 (AB, 4H, J = 8 Hz) ; 6,65 à 6,35 (s large, 1H) ; 4,05 (q, 2H, J = 6Hz) ; 2,05 (s, 3H) ; 1,30 (t, 3H, J = 6Hz).

B) Préparation de l'acide (Z)-2-bromométhyl 3-(4-éthoxy phényl) propénoïque.
On procède de façon analogue à celle décrite à l'exemple 17 (étape B).
Rendement : 45 %
F : 205°C
IR (nujol) 1665 cm⁻¹
RMN¹H (CDCl₃/TMS) : 10,40 à à 10,00 (s large, 1H) ; 7,80 (s, 1H) ; 7,50 et 6,90 (AB, 4H, J = 8Hz) ; 4,40 (s, 2H) ; 4,05 (q, 2H, J = 6Hz) ; 1,30 (t, 3H, J = 6Hz).

C) Préparation de l'acide (Z)-2-(acétylthiométhyl) 3-(4-éthoxy phényl) propénoïque.
On procède de façon analogue à celle décrite à l'exemple 17 (étape C)
Rendement : 96 %
F = 145°C
IR (nujol) : 1690, 1670 cm⁻¹
RMN¹H (CDCl₃/TMS) : 11,00 (s, 1H) ; 7,85 (s, 1H) ; 7,40 et 6,95 (AB, 4H, J = 8Hz) ; 4,25 à 3,90 (m, 4H) ; 2,35 (s, 3H) ; 1,35 (t, 3H, J = 6Hz).

D) Préparation du N-(Z)-[1-oxo 2-(acétylthiométhyl) 3-(4-éthoxy phényl) propényl]-β-alaninate de benzyle.
On couple l'acide (Z)-2-acétylthiométhyl 3-(4-éthoxy phényl) propénoïque obtenu à l'étape C avec le β-alaninate de benzyle selon le mode opératoire décrit à l'exemple 1 (étape D).
Rendement = 65 % (purification par chromatographie éclair, éluant : éther-éther de pétrole 65/35)
F = 92°C
IR (nujol) : 3290, 1740, 1690, 1640, 1620 cm⁻¹
RMN¹H (CDCl₃/TMS) : 7,45 (s, 1H) ; 7,35 à 7,15 (m, 9 H) ; 7,05 à 6,60 (m, 1H) ; 5,15 (s, 2H) ; 4,15 à 3,85 (m, 4H) ; 3,75 à 3,45 (q app., 2H, J app. : 6Hz) ; 2,65 (t, 2H, J = 7Hz) ; 2,30 (s, 3H) ; 1,35 (t,3H, J = 6Hz).

| Microanalyse : C₂₄H₂₇O₅NS | | | |
|---|---|---|---|
| Calc. % | C = 65,29 | H = 6,16 | N = 3,17 |
| Tr. % | C = 65,37 | H = 6,11 | N = 3,29 |

### Exemple 27 : Préparation du N-(E)-[1-oxo 2-(acétylthiométhyl) 3-(4-éthoxy phényl) propényl]-β-alaninate de benzyle.

On procède de façon analogue à celle décrite à l'exemple 18, en partant de l'isomère (Z) précédent.
Rendement = 42 % (après chromatographie éclair, éluant : éther-éther de pétrole 62/38)
F = 87°C
IR (nujol) 3290, 1740, 1690, 1630 cm⁻¹
RMN¹H (CDCl₃/TMS) : 7,40 à 7,00 (m, 9H) ; 6,75 (s, 1H) ; 6,05 (t large, 1H) ; 4,95 (s, 2H) ; 4,10 à 3,75 (m, 4 H) ; 3,50 (q app., 2 H, J app. = 6Hz) ; 2,60 à 2,20 (m, 5H) ; 1,30 (t, 3H, J = 7 Hz).

| Microanalyse : C₂₄H₂₇O₅NS | | | |
|---|---|---|---|
| Calc. % | C = 65,29 | H = 6,16 | N = 3,17 |
| Tr. % | C = 65,37 | H = 6,34 | N = 3,22 |

### Exemple 28 : Préparation de la N-(E)-[1-oxo 2-(mercaptométhyl) 3-(4-éthoxy phényl) propényl]-β-alanine.

On effectue la saponification du diester (E) obtenu à l'exemple 27 selon le mode opératoire décrit à l'exemple 4.
Rendement : 29 %
F = 148°C
IR (nujol) : 1730, 1620, 1580 cm⁻¹
RMN¹H (CDCl₃) : 8,30 (s large, 1H) ; 7,75 et 7,15 (AB, 4H, J = 8Hz) ; 6,55 (s, 1H) ; 6,15 (t, 1H, J = 5 Hz) ; 3,95 (q, 2H, J = 6Hz) ; 3,50 à 3,45 (m, 4H) ; 2,50 (t, 2H, J = 6Hz) ; 1,70 (t, 1H, J = 8Hz) ; 1,35 (t, 3H, J = 6Hz).

| Microanalyse : C₁₅H₁₉O₄NS | | | |
|---|---|---|---|
| Calc. % | C = 58,23 | H = 6,19 | N = 4,53 |
| Tr. % | C = 58,51 | H = 6,12 | N = 4,81 |

### Exemple 29 : Préparation du N-(Z)-[1-oxo 2-(acétylthiométhyl) 3-(4-propoxy phényl) propényl]- β -alaninate de benzyle

A) Préparation de l'acide (E)-2-méthyl 3-(4-propoxy phényl) propénoïque.
On procède de façon analogue à celle décrite à l'exemple 17 (étape A), en partant du 4-propoxybenzaldéhyde.
Rendement = 60 %
F = 171°C
IR (nujol) : 1665 cm⁻¹
RMN¹H (CDCl₃/TMS) : 9,00 à 8,30 (s large, 1H) ; 7,75 (s, 1H); 7,35 et 6,90 (AB, 4H, J = 8 Hz) ; 3,95 (t, 2H, J = 7 Hz) ; 2,15 (s, 3H) ; 1,90 à 1,65 (m, 2H) ; 1,00 (t, 3H, J = 9 Hz).

B) Préparation de l'acide (Z)-2-bromométhyl 3-(4-propoxy phényl) propénoïque.
On procède de façon analogue à celle décrite à l'exemple 17 (étape B).
Rendement : 73 %
F = 189°C
IR (nujol) : 1670 cm⁻¹
RMN¹H (CDCl₃/TMS) : 11,50 à 11,00 (s large, 1H) ; 7,80 (s, 1H) ; 7,40 et 7,00 (AB, 4H, J = 8Hz) ; 4,40 (s, 2H) ; 3,90 (t, 2H, J = 7 Hz) ; 1,80 (q. app., 2H, J app. = 7 Hz) 1,00 (t, 3H, J = 8 Hz).

C) Préparation de l'acide (Z)-2-acétylthiométhyl 3-(4-propoxy phényl) propénoïque
On procède de façon analogue à celle décrite à l'exemple 17 (étape C).
Rendement : 98 %
F = 156°C
IR (nujol) : 1690, 1675, 1615 cm⁻¹
RMN¹H (CDCl₃/TMS) : 8,60 à 8,30 (s large, 1H) ; 7,90 (s, 1H); 7,40 et 6,95 (AB, 4H, J = 8Hz) ; 4,15 (s, 2H) ; 3,95 (t, 2H, J = 7 Hz) ; 2,35 (s, 3H) ; 1,80 (q, 2H, J app. = 7 Hz) ; 1,00 (t, 3H, J = 8 Hz).

D) Préparation du N-(Z)-[1-oxo 2-(acétylthiométhyl) 3-(4-propoxy phényl) propényl]-β-alaninate de benzyle.
On couple l'acide (Z)-2-acétylthiométhyl 3-(4-propoxy phényl) propénoïque obtenu à l'étape C avec le β-alaninate de benzyle selon le mode opératoire décrit à l'exemple 1 (étape D).
Rendement = 66 % (purification par chromatographie éclair, éluant : éther-éther de pétrole 65/35) - huile
IR : 3280, 1740, 1690, 1645, 1620 cm⁻¹
RMN¹H (CDCl₃/TMS) : 7,50 (s, 1H) ; 7,35 à 7,15 (m, 7 H) ; 6,95 à 6,75 (m, 3H) ; 5,15 (s, 2H) ; 4,00 à 3,50 (m, 6 H) ; 2,65 (t, 2 H, J = 6 Hz) ; 2,30 (s, 3 H) ; 1,80 (m, 2 H, J app. = 8 Hz) ; 1,00 (t, 3H, J = 8 Hz).

| Microanalyse : C₂₅ H₂₉ O₅ NS | | | |
|---|---|---|---|
| Calc. % | C = 65,91 | H = 6,42 | N = 3,07 |
| Tr. % | C = 65,80 | H = 6,38 | N = 2,91 |

### Exemple 30 : Préparation du N-(E)-[1-oxo 2-(acétylthiométhyl) 3-(4-propoxy phényl) propényl]-β-alaninate de benzyle.

On procède de façon analogue à celle décrite à l'exemple 18, en partant de l'isomère (Z).
Rendement = 47 % (purification par chromatographie éclair, éluant : éther-éther de pétrole 60:40) - huile
IR : 3290, 1740, 1690, 1630 cm⁻¹
RMN¹H (CDCl₃/TMS) : 7,55 à 6,65 (m, 10H) ; ; 6,15 à 5,85 (m, 1H) ; 5,00 (s, 2H) ; 4,05 à 3,35 (m, 6 H) ; 2,60 à 2,30 (m, 5H) ; 1,75 (m, 2H, J app. = 7 Hz) ; 0,95 (t, 3 H, J = 7 Hz).

| Microanalyse : C₂₅H₂₉O₅NS | | | |
|---|---|---|---|
| Calc. % | C = 65,91 | H = 6,42 | N = 3,07 |
| Tr. % | C = 65,90 | H = 6,53 | N = 3,21 |

### Exemple 31 : Préparation du N-(Z)-[1-oxo 2-(acétylthiométhyl) 3-(3,5-difluoro phényl) propényl]-β-alaninate de benzyle.

A) Préparation de l'acide (E) 2-méthyl 3-(3,5-difluoro phényl) propénoïque.
On procède de façon analogue à celle décrite à l'exemple 17 (étape A), en partant du 3,5-difluoro benzaldéhyde.
Rendement = 82 %
F = 148°C
IR (nujol) : 1695, 1620, 1590 cm⁻¹
RMN¹H (CDCl₃/TMS) : 11,30 (s, 1H) ; 7,70 (s, 1H) ; 7,40 à 6,60 (m, 3H) ; 2,05 (s, 3H).

B) Préparation de l'acide (Z)-2-bromométhyl 3-(3,5-difluoro phényl) propénoïque.
On procède de façon analogue à celle décrite à l'exemple 17 (étape B)
Rendement : 63 %
F = 151°C
IR (nujol) : 1700, 1620, 1590 cm⁻¹
RMN¹H (CDCl₃/TMS) : 8,50 à 8,30 (s large, 1H) ; 7,65 (s, 1H); 7,45 à 6,75 (m, 3H) ; 4,25 (s, 2H)

C) Préparation de l'acide (Z)-2-acétylthiométhyl 3-(3,5-difluoro phényl) propénoïque.
On procède de façon analogue à celle décrite à l'exemple 17 (étape C)
Rendement : 94 %
F = 139°C
IR (nujol) : 1700, 1620, 1590 cm⁻¹
RMN¹H (CDCl₃/TMS) : 10,35 à 10,15 (s large, 1H) ; 7,85 (s, 1H) ; 7,05 à 6,95 (m, 3H) ; 4,05 (s, 2H) ; 2,30 (s, 3H).

D) Préparation du N-(Z)-[1-oxo 2-(acétylthiométhyl) 3-(3,5-difluoro phényl) propényl]-β-alaninate de benzyle.
On couple l'acide (Z) obtenu à l'étape précédente C, avec le β-alaninate de benzyle selon le mode opératoire décrit à l'exemple 1 (étape D).
Rendement : 54 % (après chromatographie éclair, éluant : éther-éther de pétrole 60/40)
F = 82°C
IR (nujol) : 3300, 1750, 1690, 1650 cm⁻¹
RMN¹H (CDCl₃/TMS) : 7,40 à 7,05 (m, 6H) ; 6,95 à 6,70 (m,4H); 5,15 (s,2H) ; 3,90 (s,2H) ; 3,65 à 3,45 (m, 2H) ; 3,65 (t, 2H , J = 6Hz) ; 2,35 (s, 3H).

| Microanalyse : C₂₂ H₂₁ O₄ N S F₂ | | | |
|---|---|---|---|
| Calc. % | C = 60,96 | H = 4,88 | N = 3,23 |
| Tr. % | C = 60,78 | H = 4,77 | N = 3,01 |

### Exemple 32 : Préparation du N-(E)-[1-oxo Z-(acétylthiométhyl) 3-(3,5-difluoro phényl) propényl]-β-alaninate de benzyle

On procède de façon analogue à celle décrite à l'exemple 18, en partant de l'isomère (Z) précédent.
Rendement : 61 % (après chromatographie éclair, éluant : éther-éther de pétrole 55/45)
F : 81°C
IR (nujol) : 3300, 1750, 1690, 1640, 1620 cm⁻¹
RMN¹H (CDCl₃) : 7,35 à 7,25 (m, 5H) ; 6,80 (d, 2H, J app. = 10 Hz) ; 6,65 (s, 1H) ; 6,60 (d, 1H, J app. = 10 Hz) ; 6,10 (t, 1H, J = 5Hz) ; 5,00 (s, 2H) ; 3,80 (s, 2H) ; 3,45 (q, 2H, J app. = 6Hz) ; 2,50 (t, 2H, J = 6Hz) ; 2,30 (s, 3H).

| Microanalyse : C₂₂ H₂₁ O₄ N S F₂ | | | |
|---|---|---|---|
| Calc. % | C = 60,96 | H = 4,88 | N = 3,23 |
| Tr. % | C = 60,80 | H = 4,70 | N = 3,03 |

### Exemple 33 : Préparation de la N-(E)-[1-oxo 2-(mercaptométhyl) 3-(3,5-difluoro phényl) propényl]-β-alanine

On effectue la saponification du diester (E) obtenu à l'exemple 32 selon le mode opératoire décrit à l'exemple 4.
Rendement : 27 %
F : 125°C
IR (nujol) : 1725, 1630, 1580 cm⁻¹
RMN¹H (CDCl₃) : 9,40 (s large, 1 H) ; 6,80 à 6,65 (m, 3H) ; 6,55 (s, 1H) ; 6,15 (t large, 1H) ; 3,55 à 3,40 (m, 4H) ; 2,50 (t, 2H, J = 5 Hz) ; 1,70 (t, 1H, J = 8Hz).

| Microanalyse : C₁₃ H₁₃ O₃ N S F₂ | | | |
|---|---|---|---|
| Calc. % | C = 51,82 | H : 4,35 | N = 4,65 |
| Tr. % | C = 52,11 | H : 4,26 | N = 4,42 |

### Exemple 34 : Préparation du N-(Z)-[1-oxo 2-(acétylthiométhyl) 3-(1-naphtyl) propényl]-β-alaninate de benzyle

A) Préparation de l'acide (E) 2-méthyl 3-(1-naphtyl) propénoïque.
On procède de façon analogue à celle décrite à l'exemple 17 (étape A), en partant du 1-naphtaldéhyde.
Rendement : 65 %
F : 155°C
IR (nujol) : 1670 cm⁻¹
RMN¹H (CDCl₃/TMS) : 11,40 à 10,80 (s large, 1 H) ; 8,20 (s, 1 H) ; 8,00 à 7,70 (m, 3H) ; 7,65 à 7,20 (m, 4 H) ; 2,00 (s, 3H).

B) Préparation de l'acide (Z)-2-bromométhyl 3-(1-naphtyl) propénoïque.
On procède de façon analogue à celle décrite à l'exemple 17 (étape B).
Rendement : 50 %
F = 216°C
IR (nujol) : 1670 cm⁻¹
RMN¹H (CDCl₃/TMS) : 8,30 (s, 1H) ; 8,05 à 7,80 (m, 3H) ; 7,75 à 7,40 (m, 4H) ; 6,30 à 5,80 (s large, 1H) ; 4,30 (s, 2H).

C) Préparation de l'acide (Z)-2-acétylthiométhyl 3-(1-naphtyl) propénoïque.
On procède de façon analogue à celle décrite à l'exemple 17 (étape C).
Rendement = 97 %
F = 175°C
IR (nujol) : 1690, 1670 cm⁻¹
RMN¹H (CDCl₃/TMS) : 10,40 à 10,00 (s large, 1H) ; 7,95 (s, 1H) ; 7,75 à 7,20 (m, 9H) ; 4,15 (s, 2H) ; 2,35 (s, 3H)

D) Préparation du N-(Z)-[1-oxo-2-(acétylthiométhyl) 3-(1-naphtyl) propényl]-β-alaninate de benzyle.
On couple l'acide (Z) précédent avec le β-alaninate de benzyle selon le mode opératoire décrit à l'exemple 1 (étape D).
Rendement : 57 % (après chromatographie éclair, éluant : éther-éther de pétrole 60/40)
F : 81°C
IR (nujol) : 3290, 1740, 1695, 1640, 1620 cm⁻¹
RMN¹H (CDCl₃/TMS) : 8,20 (s, 1H) ; 8,00 à 7,70 (m, 3H) ; 7,65 à 7,00 (m, 10 H) ; 5,20 (s, 2H) ; 3,90 (s, 2H) ; 3,65 à 3,45 (m, 2H) ; 2,65 (t,2H, J = 6Hz) ; 2,35 (s, 3H).

| Microanalyse : C₂₆ H₂₅ O₄N S | | | |
|---|---|---|---|
| Calc. % | C = 69,78 | H = 5,63 | N = 3,13 |
| Tr. % | C = 69,86 | H = 5,68 | N = 3,17 |

### Exemple 35 : Préparation du N-(E)-[1-oxo 2-(acétylthiométhyl) 3-(1-naphtyl) propényl]-β-alaninate de benzyle

On procède de façon analogue à celle décrite à l'exemple 18, en partant de l'isomère (Z).
Rendement : 51 % (après chromatographie éclair, éluant : éther-éther de pétrole 57/43)
F = 78°C
IR (nujol) : 3290, 1740, 1695, 1630 cm⁻¹
RMN¹H (CDCl₃/TMS) : 7,95 à 7,75 (m, 3H) ; 7,55 à 7,10 (m, 10H) ; 5,80 à 5,70 (m, 1H) ; 5,00 (s, 2H) ; 4,05 (s, 2H) ; 3,65 à 3,45 (m, 2H) ; 2,65 (t, 2H, J = 6Hz) ; 2,35 (s, 3H).

| Microanalyse : C₂₆ H₂₅ O₄ N S | | | |
|---|---|---|---|
| Calc. % | C = 69,78 | H = 5,63 | N = 3,13 |
| Tr. % | C = 69,48 | H = 5,71 | N = 3,01 |

### Exemple 36 : Préparation de la N-(E)-[1-oxo 2-(mercaptométhyl) 3-(1-naphtyl) propényl]-β-alanine

On effectue la saponification du diester (E) obtenu à l'exemple 35 selon le mode opératoire décrit à l'exemple 4.
Rendement : 39 %
F : 133°C
IR (nujol) : 1735, 1620, 1575 cm⁻¹
RMN¹H (CDCl₃/TMS) : 8,00 à 7,75 (m, 3H) ; 7,55 à 7,10 (m, 6 H) ; 6,30 à 6,20 (m, 1H) ; 3,55 à 3,45 (m, 4H) ; 2,50 (t, 2H, J = 6Hz) ; 1,70 (t, 1H, J = 8Hz)

| Microanalyse : C₁₇ H₁₇ O₃ N S | | | |
|---|---|---|---|
| Calc. % | C = 64,74 | H = 5,43 | N = 4,44 |
| Tr. % | C = 64,47 | H = 5,29 | N = 4,31 |

### Exemple 37 : Préparation du N-(Z)-[1-oxo-2-(acétylthiométhyl) 3-phényl propényl]-β-alaninate de benzyle

On couple l'acide (Z) 2-acétylthiométhyl 3-phényl propénoïque décrit à l'exemple 1 (étape B) avec le β-alaninate de benzyle selon le mode opératoire décrit à l'exemple 1 (étape D)
Rendement : 78 % (après recristallisation dans l'éther)
F : 80°C
IR : 3380, 1720, 1670, 1630, 1600 cm⁻¹
RMN¹H (CDCl₃) : 7,60 (s, 1H) ; 7,45 à 7,15 (m, 10H) ; 6,90 (t large, 1H) ; 5,15 (s, 2H) ; 3,95 (s, 2H) ; 3,65 (q. app., J : 6,1 Hz) ; 2,65 (t, 2H, J = 6, 1 Hz) ; 2,35 (s, 3H).

| Microanalyse : C₂₂ H₂₃ NO₄ S | | | |
|---|---|---|---|
| Calc. % | C : 66,48 | H : 5,83 | N : 3,52 |
| Tr. % | C = 66,29 | H : 5,78 | N : 3,51 |

### Exemple 38 : Préparation de la N-(Z)-[1-oxo 2-(mercaptométhyl) 3-phényl propényl]-β-alanine

On place dans un ballon 0,795 g (2,0 mmol) du composé obtenu à l'étape précédente, en solution dans 6 ml de méthanol. On purge avec de l'argon et on refroidit la solution par un bain de glace. On ajoute à environ 5°C, 5 ml d'une solution aqueuse de soude 1N. On agite 2 heures à 20°C. On évapore le méthanol sous vide à une température inférieure à 35°C. La phase aqueuse basique est lavée à l'éther (2 fois 10 ml). Elle est ensuite acidifiée par une solution aqueuse d'HCl 1N jusqu'à pH 1. On l'extrait à l'éther (2 fois 10 ml). Les phases d'extraction sont lavées une fois à l'eau, séchées sur MgSO₄, filtrées et concentrées sous vide. Le résidu est séché au dessicateur sur pentaoxyde de phosphore pour éliminer l'acide acétique. On purifie par chromatographie sur silice (éluant : éther)
Rendement : 81 %
F : 132°C
IR : 3400, 1680, 1620, 1590 cm⁻¹
RMN¹H (acétone D6) : 10,8 (s large, 1H) ; 7,65 (t large, 1H); 7,60 à 7,20 (m, 5H) ; 7,20 (s, 1H) ; 3,70 à 3,45 (m, 4H) ; 2,60 (t, 2H, J = 6,7 Hz) ; 2,30 (t, 1H, J = 5,9 Hz).

| Microanalyse : C₁₃ H₁₅ NO₃ S | | | |
|---|---|---|---|
| Calc. % | C : 58,85 | H : 5,70 | N : 5,28 |
| Tr. % | C : 58,62 | H : 5,72 | N : 5,21 |

### Exemple 39 : Préparation du N-(Z)-[1-oxo 2-(acétylthiométhyl) 3-(4-phénoxy phényl) propényl]-β-alaninate de benzyle.

A) Préparation de l'acide (E) 2-méthyl 3-(4-phénoxy phényl) propénoïque.
On procède de façon analogue à celle décrite à l'exemple 17 (étape A), en partant du 4-phénoxy benzaldéhyde et en utilisant 5 équivalents d'anhydride propionique.
Rendement : 68 %
IR (nujol) : 1665 cm⁻¹
F : 133°C
RMN¹H (CDCl₃/TMS) : 11,95 (s large, 1H) ; 7,80 (s, 1H) ; 7,60 à 6,85 (m, 9H) ; 2,15 (s, 3H).

B) Préparation de l'acide (Z)-2-bromométhyl 3-(4-phénoxy phényl) propénoïque.
On procède de façon analogue à celle décrite à l'exemple 17 (étape B).
Rendement :76 %
F : 126°C
IR (nujol) : 1670 cm⁻¹
RMN¹H (CDCl₃/TMS) : 8,60 (s large, 1H) ; 7,75 (s, 1H) ; 7,55 à 6,85 (m, 9H) ; 4,40 (s, 2H).

C) Préparation de l'acide (Z)-2-acétylthiométhyl 3-(4-phénoxy phényl) propénoïque.
On procède de façon analogue à celle décrite à l'exemple 17 (étape C).
Rendement : 100 %
F : 118°C
IR (nujol) : 1690, 1675, 1615 cm⁻¹
RMN¹H (CDCl₃/TMS) : 10,30 (s, 1H) ; 7,80 (s, 1H) ; 7,55 à 6,85 (m, 9H) ; 3,80 (s, 2H) ; 2,30 (s, 3H).

D) Préparation du N-(Z)-[1-oxo 2-(acétylthiométhyl) 3-(4-phénoxy phényl) propényl]-β-alaninate de benzyle.
On couple l'acide (Z) précédent avec le β-alaninate de benzyle selon le mode opératoire décrit à l'exemple 1 (étape D).
Rendement : 66 % (après chromatographie éclair, éluant : acétate d'éthyle-éther de pétrole 30/70)
F = 79°C
IR (nujol) : 1730, 1690, 1645 cm⁻¹
RMN¹H (CDCl₃) : 7,50 (s, 1H) ; 7,40 à 7,00 (m, 14 H) ; 7,00 (t, 1H, J = 6 Hz) ; 5,10 (s, 2H) ; 4,00 (s, 2H) ; 3,60 (q. app., 2H, J app. = 7Hz) ; 2,65 (t, 2H, J = 6Hz) ; 2,35 (s, 3H).
RMN¹³C (CDCl₃) : 196,4 ; 172,4 ; 167,2 ; 158,3 ; 156,4 ; 138,0 ; 136,0 ; 131,4 ; 130,2 ; 129,7 ; 129,6 ; 128,9 ; 128,6 ; 128,5 ; 124,3 ; 119,9 ; 118,5 ; 66,7 ; 35,9 ; 34,2 ; 30,6 ; 26,8.

| Microanalyse : C₂₈ H₂₇ O₅ N S | | | |
|---|---|---|---|
| Calc. % | C = 68,69 | H = 5,56 | N = 2,86 |
| Tr. % | C = 68,76 | H = 5,68 | N = 2,97 |

### Exemple 40 : Préparation du N-(E)-[1-oxo 2-(acétylthiométhyl) 3-(4-phénoxy phényl) propényl]-β-alaninate de benzyle

On procède de façon analogue à celle décrite à l'exemple 18, en partant de l'isomère (Z) de l'exemple 39 (étape D).
Rendement : 73 % (après chromatographie éclair, éluant : acétate d'éthyle-éther de pétrole 25/75).
F : 88°C
IR (nujol) : 1730, 1705, 1625 cm⁻¹
RMN¹H (CDCl₃) : 7,30 à 6,90 (m, 14 H) ; 6,70 (s, 1H) ; 6,10 (t, 1H, J = 6Hz) ; 5,00 ( s, 2H) ; 3,80 (s, 2H) ; 3,50 (q. app., 2H, J app. = 6Hz) ; 2,50 (t, 2H, J = 6Hz) ; 2,30 (s, 3H).
RMN¹³C (CDCl₃) : 194,9 ; 171, 8 ; 168,4 ; 157,4 ; 156,4 ; 135,4 ; 133,2 ; 131,4 ; 130,0 ; 129,8 ; 129,7 ; 128,6 128,3 ; 128,2 ; 123,6 ; 119,1 ; 118,3 ; 66,4 ; 34,6 ; 33,6 ; 33,3 ; 30,5.

| Microanalyse : C₂₈ H₂₇ O₅ N S | | | |
|---|---|---|---|
| Calc. % | C : 68,69 | H : 5,56 | N : 2,86 |
| Tr. % | C : 68,72 | H : 5,58 | N : 2,79 |

### Exemple 41 : Préparation de la N-(E)-[1-oxo 2-(mercaptométhyl) 3-(4-phénoxy phényl) propényl]-β-alanine

On effectue la saponification du diester (E) obtenu à l'exemple 40 selon le mode opératoire décrit à l'exemple 4.
Rendement : 55 %
F = 108°C
RMN¹H (CDCl₃) : 9,60 (s large, H) ; 7,30 à 6,80 (m, 9H) ; 6,50 (s, 1H) ; 6,30 (t, 1H, J = 6Hz) ; 3,50 à 3,40 (m, 4H) ; 2,50 (t, 2H, J = 6Hz) ; 1,70 (t, 1H, J = 8Hz). IR (nujol) : 3290, 1725, 1660, 1650 cm⁻¹

| Microanalyse : C₁₉ H₁₉ N S O₄ | | | |
|---|---|---|---|
| Calc. % | C : 63,85 | H : 5,38 | N : 3,92 |
| Tr. % | C : 63,66 | H : 5,14 | N : 3,69 |

### Exemple 42 : Préparation du N-(Z)-[1-oxo 2-(acétylthiométhyl) 3-(3,4-méthylènedioxy phényl) propényl]-β-alaninate de benzyle

A) Préparation de l'acide (E) 2-méthyl 3-(3,4-méthylènedioxy phényl) propénoïque.
On procède de façon analogue à celle décrite à l'exemple 17 (étape A), en partant du pipéronal.
Rendement: 65 %
F : 205°C
RMN¹H (DMSOD₆) : 10,1 (s large, 1H) ; 7,50 (s, 1H) ; 7,00 (s, 1H) ; 6,95 (s, 2H) ; 6,05 (s, 2H) ; 2,00 (s, 3H).

B) Préparation de l'acide (Z)-2-bromométhyl 3-(3,4-méthylènedioxy phényl) propénoïque.
On procède de façon analogue à celle décrite à l'exemple 17 (étape B).
Rendement : 75 %
F : 158°C
RMN¹H (CDCl₃/TMS) : 8,60 (s, 1H) ; 7,70 (s, 1H) ; 7,30 à 6,70 (m, 3H) ; 6,00 (s, 2H) ; 4,35 (s, 2H).

C) Préparation de l'acide (Z)-2-acétylthiométhyl 3-(3,4-méthylènedioxy phényl) propénoïque.
On procède de façon analogue à celle décrite à l'exemple 17 (étape C).
Rendement : 86 %
F : 142°C
RMN¹H (CDCl₃/TMS) : 8,60 (s, 1H) ; 7,80 (s, 1H) ; 7,10 à 6,70 (m, 3H) ; 6,00 (s, 2H) ; 4,05 (s, 2H) ; 2,30 (s, 3H).

D) Préparation du N-(Z)-[1-oxo 2-(acétylthiométhyl) 3-(3,4-méthylènedioxy phényl) propényl]-β-alaninate de benzyle.
On couple l'acide (Z) précédent avec le β-alaninate de benzyle selon le mode opératoire décrit à l'exemple 1 (étape D).
Rendement : 61 % (après chromatographie éclair, éluant : éther-éther de pétrole 50/50).
F = 76°C
IR (nujol) : 1730, 1705, 1620 cm⁻¹
RMN¹H (CDCl₃) : 7,55 (s, 1H) ; 7,35 (s, 5H) ; 6,90 (s large, 1H) ; 6,80 (s large, 3H) ; 6,00 (s, 2H) ; 5,20 (s, 2H) ; 4,00 (s, 2H) ; 3,65 (q. app., 2H, J. app = 7Hz) ; 2,70 (t, 2H, J = 6Hz) ; 2,35 (s, 3H).

| Microanalyse : C₂₃ H₂₃ O₆ N S | | | |
|---|---|---|---|
| Calc. % | C : 62,57 | H : 5,25 | N : 3,17 |
| Tr. % | C : 62,76 | H : 5,48 | N : 3,23 |

### Exemple 43 : Préparation du N-(E)-[1-oxo 2-(acétylthiométhyl) 3-(3,4-méthylènedioxy phényl) propényl]-β-alaninate de benzyle

On procède de façon analogue à celle décrite à l'exemple 18, en partant de l'isomère (Z) de l'exemple 42 (étape D).
Rendement : 73 % (après chromatographie éclair, éluant : éther-éther de pétrole 55/45).
F : 84°C
IR (nujol) : 1735, 1695 cm⁻¹
RMN¹H (CDCl₃) : 7,30 (s large, 5 H) ; 6,95 à 6,80 (m, 4H) ; 6,20 (m, 1H) ; 5,90 (s, 2H) ; 5,10 (s, 2H) ; 3,85 (s, 2H) ; 3,50 (q. app., 2H, J app. = 6Hz) ; 2,50 (t, 2H, J = 6Hz) ; 2,30 (s, 3H).

| Microanalyse : C₂₃ H₂₃ O₆ N S | | | |
|---|---|---|---|
| Calc. % | C : 62,57 | H : 5,25 | N : 3,17 |
| Tr. % | C : 62,48 | H : 5,06 | N : 3,25 |

Les résultats des études biologiques, rapportées ci-après, mettent en évidence les propriétés inhibitrices de l'enképhalinase des composés de formule (Ia) et (Ib) conformes à l'invention.

La présente invention concerne donc également les compositions pharmaceutiques qui contiennent, à titre de principe actif, les composés de formule (Ia) ou (Ib) conformes à l'invention en quantité thérapeutiquement efficaces.

### ETUDE BIOLOGIQUE

On a procédé au dosage de l'activité inhibitrice de l'enképhalinase (J. Pharmac. Exp. Ther., 1987, 243, 666) des composés (Ia) et (Ib).

On a rassemblé dans le tableau qui suit les résultats obtenus :

### RESULTATS BIOLOGIQUES IN VITRO SUR L'ENKEPHALINASE.

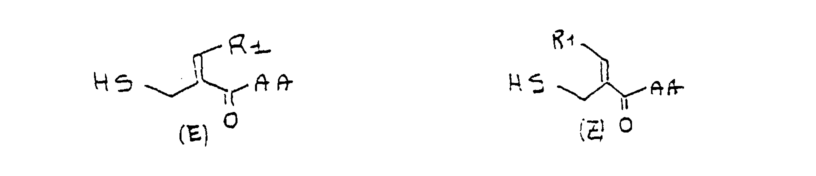

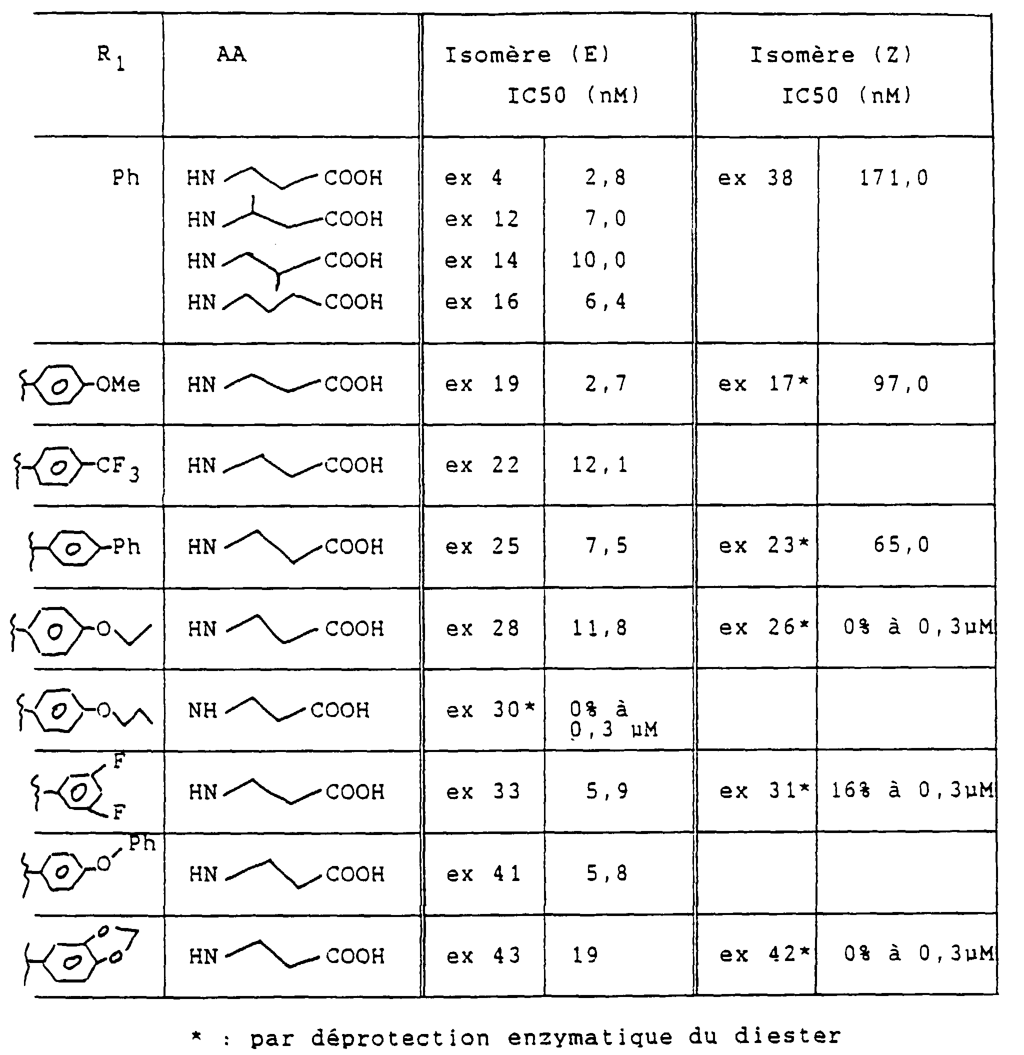

Ces résultats illustrent les intéressantes propriétés inhibitrices de l'enképhalinase des composés selon l'invention, qui les rendent utiles en médecine humaine et vétérinaire.

Les compositions pharmaceutiques qui contiennent les composés de formule (Ia) ou (Ib) selon l'invention sont utiles dans les indications résultant des propriétés centrales, notamment comme antalgiques, et périphériques, notamment comme antidiarrhéiques, des inhibiteurs de l'enképhalinase, ainsi que dans les indications des protecteurs de l'ANF, notamment l'hypertension artérielle et l'insuffisance cardiaque.

Les compositions pharmaceutiques qui contiennent, à titre de principe actif, les composés de formule (Ia) ou (Ib) conformes à l'invention, sont administrables à l'homme par voie orale, parentérale ou rectale.

Ces compositions pharmaceutiques peuvent être sous forme solide ou liquide et se présenter sous les formes pharmaceutiques couramment utilisées en médecine humaine comme, par exemple, sous forme de comprimés simples ou dragéifiés, de gélules, de suppositoires, de préparations injectables.

Les compositions pharmaceutiques conformes à l'invention sont administrables à des doses unitaires, de préférence de 20 à 200 mg de principe actif.

## Revendications

1. Dérivés d'amino-acides, caractérisés en ce qu'ils répondent aux formules générales 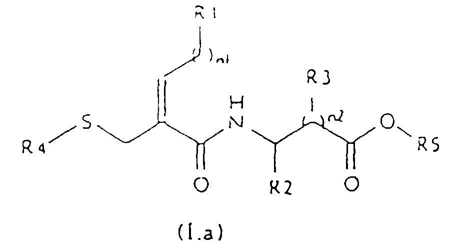 et 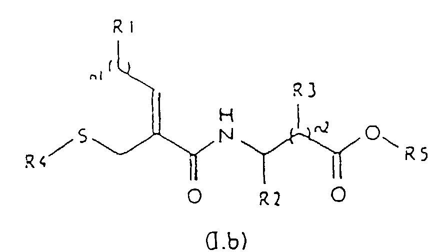 dans lesquelles R₁ représente un atome d'hydrogène, un groupe phényle, éventuellement mono- ou polysubstitué par un atome d'halogène, un groupe trifluorométhyle, un groupe nitro, un groupe cyano ou un groupe amino, un groupe alkyle inférieur, un groupe phénylalkylène inférieur ; le groupement 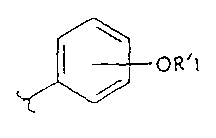 où R'₁ représente un atome d'hydrogène, un groupe alkyle inférieur, un groupe phényle, un groupe phénylalkylène inférieur ; un groupement 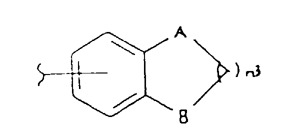 où A, B et n₃ ont la signification donnée ci-dessous
| A | B | n₃ |
|---|---|---|
| 0 | 0 | 1 |
| 0 | CH₂ | 1 |
| CH₂ | CH₂ | 1 |
| 0 | 0 | 2 |
| CH₂ | CH₂ | 2 |
| 0 | CH₂ | 2 |
un groupe biphényle, alpha et bêta naphtyle,
n₁ varie de 0 à 10
n₂ varie de 1 à 10
R₂ représente un atome d'hydrogène ; un groupe alkyle inférieur ; un groupe hydroxyalkylène inférieur ; un groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inférieur ; un groupe alkyle inférieur thioalkylène inférieur ; un groupe imidazolylalkylène inférieur ; un groupe indolylalkylène inférieur ; un groupe carbamylalkylène inférieur ; un groupe carboxyalkylène inférieur ;
R₃ représente également un atome d'hydrogène ou un groupe alkyle inférieur ;
R₄ représente un atome d'hydrogène, un radical acyle aliphatique linéaire ou ramifié, un radical acyle aromatique éventuellement mono- ou polysubstitué ou un radical acyle linéaire ou ramifié comportant un ou plusieurs atomes d'oxygène ;
R₅ représente un atome d'hydrogène ; un groupe alkyle inférieur linéaire ou ramifié ; un groupe phényle, un groupe phénylalkylène inférieur, ces deux derniers groupes étant éventuellement mono- ou polysubstitués sur le noyau phényle; un substituant linéaire ou ramifié comportant un ou plusieurs atomes d'oxygène,
les termes "alkyle inférieur" ou "alkylène inférieur" désignant respectivement un groupe alkyle ou alkylène à chaîne linéaire ou ramifiée contenant 1 à 6 atomes de carbone, de préférence 1 à 4 atomes de carbone.

2. Dérivés d'amino-acides selon la revendication 1, caractérisés en ce que R₁ représente un groupe phényle éventuellement mono- ou polysubstitué par un atome d'halogène, par le groupe trifluorométhyle ; le groupement 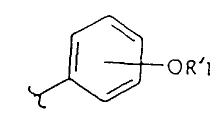 où R'₁ représente un groupe alkyle inférieur, un groupe phényle ; le groupement 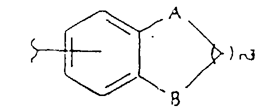 où A et B représentent l'oxygène et n₃ est égal à 1 ou 2 ; un groupe biphényle, alpha et bêta naphtyle ;
R₂ et R₃ représentent un atome d'hydrogène, un radical alkyle inférieur ;
R₄ représente un atome d'hydrogène, un radical acyle aliphatique linéaire ou ramifié ou un radical acyle aromatique;
R₅ représente un atome d'hydrogène, un radical alkyle inférieur, un radical phényle ;
n₁ est égal à 0 ou 1 ;
n₂ est égal à 1 ou 2.

3. Dérivés d'amino-acides selon la revendication 1, caractérisés en ce qu'ils sont choisis parmi :
- le N-(E)-[1-oxo 2-(acétylthiométhyl) 3-phényl propényl]-β-alaninate de benzyle,
- le N-(E)-[1-oxo 2-(acétylthiométhyl) 3-phényl propényl]-β-alaninate de méthyle,
- le N-(E)-[1-oxo 2-(acétylthiométhyl) 3-phényl propényl]-β-alaninate d'éthyle,
- la N-(E)-[1-oxo 2-(mercaptométhyl) 3-phényl propényl]-β-alanine,
- le N-(E)-[1-oxo 2-(benzoylthiométhyl) 3-phényl propényl]-β-alaninate de benzyle,
- le N-(E)-[1-oxo 2-(benzoylthiométhyl) 3-phényl propényl]-β-alaninate de méthyle,
- le N-(E)-[1-oxo 2-(benzoylthiométhyl) 3-phényl propényl]-β-alaninate d'éthyle,
- le N-(E)-[1-oxo 2-(pivaloylthiométhyl) 3-phényl propényl]-β-alaninate de benzyle,
- le N-(E)-[1-oxo 2-(pivaloylthiométhyl) 3-phényl propényl]-β-alaninate de méthyle,
- le N-(E)-[1-oxo 2-(pivaloylthiométhyl) 3-phényl propényl]-β-alaninate d'éthyle,
- le N-(E)-[1-oxo 2-(acétylthiométhyl) 3-phényl propényl]-(RS)-3-amino butanoate de méthyle,
- l'acide N-(E)-[1-oxo 2-(mercaptométhyl) 3-phényl propényl]-(RS)-3-amino butanoïque,
- le N-(E)-[1-oxo 2-(acétylthiométhyl) 3-phényl propényl]-(RS)-3-amino 2-méthyl propanoate de méthyle,
- l'acide N-(E)-[1-oxo 2-(mercaptométhyl) 3-phényl propényl]-(RS)-3-amino 2-méthyl propanoïque,
- le N-(E)-[1-oxo 2-(acétylthiométhyl) 3-phényl propényl]-4-amino butanoate de benzyle,
- l'acide N-(E)-[1-oxo 2-(mercaptométhyl) 3-phényl propényl]-4-amino butanoïque,
- le N-(Z)-[1-oxo 2-(acétylthiométhyl) 3-(4-méthoxy phényl) propényl]-β-alaninate de benzyle,
- le N-(E)-[1-oxo 2-(acétylthiométhyl) 3-(4-méthoxy phényl) propényl]-β- alaninate de benzyle,
- la N-(E)-[1-oxo 2-(mercaptométhyl) 3-(4-méthoxy phényl) propényl]-β-alanine,
- le N-(Z)-[1-oxo 2-(acétylthiométhyl) 3-(4-trifluorométhyl phényl) propényl] β-alaninate de benzyle,
- le N-(E)-[1-oxo 2-(acétylthiométhyl) 3-(4-trifluorométhyl phényl) propényl]-β-alaninate de benzyle,
- la N-(E)-[1-oxo 2-(mercaptométhyl) 3-(4-trifluorométhyl phényl) propényl]-β-alanine,
- le N-(Z)-[1-oxo 2-(acétylthiométhyl) 3-(4-phényl phényl) propényl]-β-alaninate de benzyle,
- le N-(E)-[1-oxo 2-(acétylthiométhyl) 3-(4-phényl phényl) propényl]-β-alaninate de benzyle,
- la N-(E)-[1-oxo 2-(mercaptométhyl) 3-(4-phényl phényl) propényl]-β-alanine,
- le N-(Z)-[1-oxo 2-(acétylthiométhyl) 3-(4-éthoxy phényl) propényl]-β-alaninate de benzyle,
- le N-(E)-[1-oxo 2-(acétylthiométhyl) 3-(4-éthoxy phényl) propényl]-β-alaninate de benzyle,
- la N-(E)-[1-oxo 2-(mercaptométhyl) 3-(4-éthoxy phényl) propényl]-β-alanine,
- le N-(Z)-[1-oxo 2-(acétylthiométhyl) 3-(4-propoxy phényl) propényl]-β-alaninate de benzyle,
- le N-(E)-[1-oxo 2-(acétylthiométhyl) 3-(4-propoxy phényl) propényl]-β-alaninate de benzyle,
- le N-(Z)-[1-oxo 2-(acétylthiométhyl) 3-(3,5-difluoro phényl) propényl]-β-alaninate de benzyle,
- le N-(E)-[1-oxo 2-(acétylthiométhyl) 3-(3,5-difluoro phényl) propényl]-β-alaninate de benzyle,
- la N-(E)-[1-oxo 2-(mercaptométhy!) 3-(3,5-difluorophényl) propényl]-β-alanine,
- le N-(Z)-[1-oxo 2-(acétylthiométhyl) 3-(1-napthyl) propényl]-β-alaninate de benzyle,
- le N-(E)-[1-oxo 2-(acétylthiométhyl) 3-(1-napthtyl) propényl]-β-alaninate de benzyle,
- la N-(E)-[1-oxo 2-(mercaptométhyl) 3-(1-naphtyl) propényl]-β-alanine,
- le N-(Z)-[1-oxo 2-(acétylthiométhyl) 3-phényl propényl]-β-alaninate de benzyle,
- la N-(Z)-[1-oxo 2-(mercaptométhyl) 3-phényl propényl]-β-alanine,
- le N-(Z)-[1-oxo 2-(acétylthiométhyl) 3-(4-phénoxy phényl) propényl]-β-alaninate de benzyle,
- le N-(E)-[1-oxo 2-(acétylthiométhyl) 3-(4-phénoxy phényl) propényl]-β-alaninate de benzyle,
- la N-(E)-[1-oxo 2-(mercaptométhyl) 3-(4-phénoxy phényl) propényl]-β-alanine,
- le N-(Z)-[1-oxo 2-(acétylthiométhyl) 3-(3,4-méthylènedioxy phényl) propényl]-β-alaninate de benzyle,
- le N-(E)-[1-oxo 2-(acétylthiométhyl) 3-(3,4-méthylènedioxy phényl) propényl]-β-alaninate de benzyle.

4. Procédé de préparation des dérivés d'amino-acides de formule (Ib) dans laquelle R₁, R₂, R₃, R₄, R₅, n₁, n₂ et n₃ ont les significations données dans la revendication 1, caractérisé en ce qu'il consiste successivement :
a) à effectuer une bromation allylique d'un acide éthylénique de configuration (E) de formule (II) 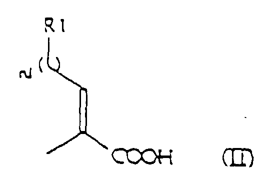 dans laquelle R₁ a la signification précitée, avec un agent de bromation tel que le N-bromosuccinimide en présence d'une quantité catalytique de peroxyde de benzoyle pour former un acide de formule (III) 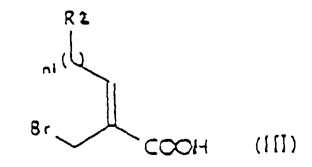
b) à substituer le brome de l'acide de formule (III), de préférence par un thioacide R₄-SH, pour former l'acide éthylénique thioacylé de configuration (Z) de formule (IV) 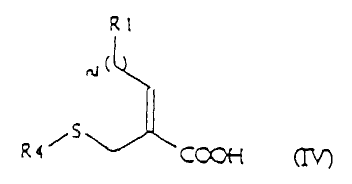 où R₄ a la définition donnée dans la revendication 1,
c) à coupler l'acide de formule (IV) avec le sel d'amino-ester désiré de formule (V) 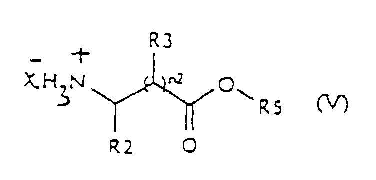 où R₂, R₃, R₅ et n₂ ont les significations données dans la revendication 1 et X représente par exemple les halogénures, le benzènesulfonate, le méthanesulfonate et le toluènesulfonate, pour former le composé de configuration (Z) de formule (Ib) 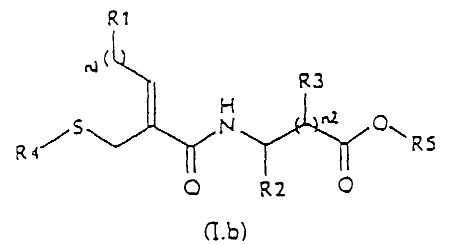 où R₄ et R₅ ne sont pas des atomes d'hydrogène,
d) à soumettre le composé de formule (Ib) à une déprotection alcaline pour former les composés de configuration (Z) de formule (Ib) où R₄ et R₅ sont des atomes d'hydrogène

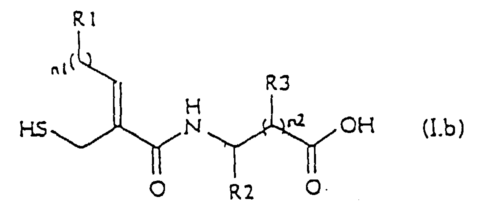

5. Procédé de préparation des dérivés d'amino-acides de formule (Ia) où R₁, R₂, R₃, R₄, R₅, n₁, n₂ et n₃ ont les significations données dans la revendication 1, caractérisé en ce qu'il consiste successivement :
a) à isomériser l'acide éthylénique de configuration (Z) de formule (IV) 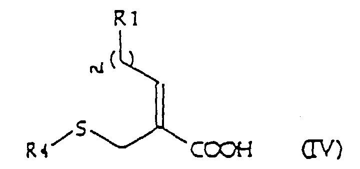 où R₁, R₄ et n₁ ont les significations données dans la revendication 1, puis à séparer le mélange d'isomères (E/Z) obtenu, par une amine telle que la cyclohexylamine, pour obtenir l'acide éthylénique thioacylé de configuration (E) de formule (VI) 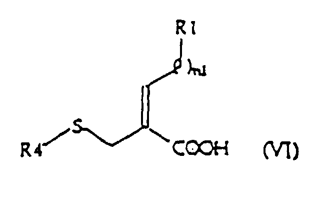
b) à coupler l'acide de formule (VI) avec le sel d'amino-ester désiré de formule (V), en présence d'un agent de couplage tel que le dicyclohexylcarbodiimide, pour obtenir le composé de formule (Ia) de configuration (E) où R₄ et R₅ ne sont pas des atomes d'hydrogène 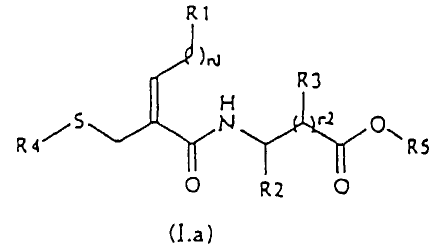
c) puis à soumettre le composé de formule (Ia) à une déprotection alcaline pour obtenir les composés de configuration (E) de formule (Ia) où R₄ et R₅ sont des atomes d'hydrogène

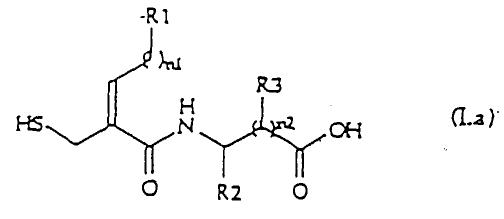

6. Procédé de préparation des dérivés d'amino-acides de formule (Ia) où R₁, R₂, R₃, R₄, R₅, n₁, n₂ et n₃ ont les significations données dans la revendication 1, caractérisé en ce qu'il consiste successivement
a) à isomériser l'acide éthylénique de formule (IV) possédant une configuration (Z) pour obtenir l'acide thioacylé éthylénique sous forme d'un mélange d'isomères de configuration (Z/E) de formule (VII) 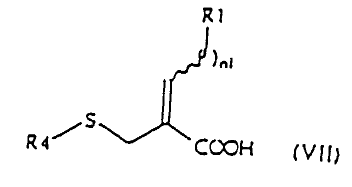 où R₁, R₄et n₁ ont les significations données dans la revendication 1,
b) à coupler l'acide de formule (VII) avec le sel d'amino-ester désiré de formule (V), en présence d'un agent de couplage tel que le dicyclohexylcarbodiimide, pour obtenir le composé de formule (VIII) constitué d'un mélange d'isomères (Z/E) 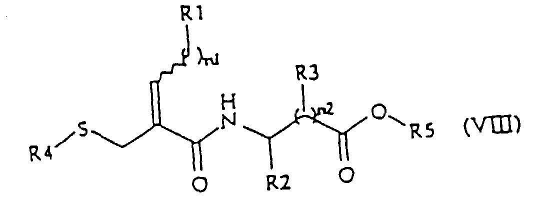
c) à séparer le mélange d'isomères (Z/E) du composé de formule (VIII), par exemple par une chromatographie éclair sur silice ou par recristallisation fractionnée, pour obtenir le composé de configuration (E) de formule (Ia) où R₄ et R₅ ne sont pas des atomes d'hydrogène

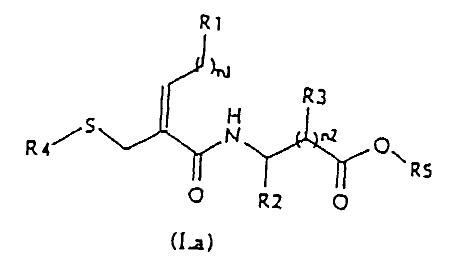

7. Procédé de préparation des dérivés d'amino-acides de formule (Ia) où R₁, R₂, R₃, R₄, R₅, n₁, n₂ et n₃ ont les significations données dans la revendication 1, R4 et R5 n'étant pas des atomes d'hydrogène, caractérisé en ce qu'il consiste successivement :
a) à isomériser le composé de formule (Ib) possédant une double liaison de configuration (Z) 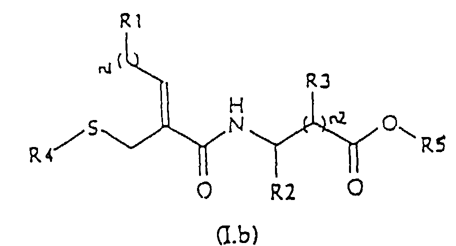 de préférence en présence d'éthérate de trifluorure de bore, pour obtenir le composé de formule (VIII) constitué d'un mélange d'isomères (Z/E) 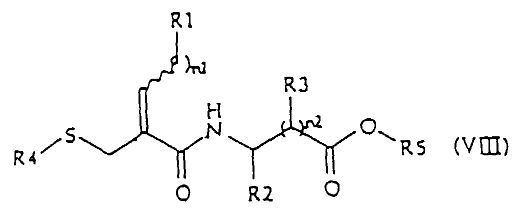
b) à séparer le mélange d'isomères (Z/E) du composé de formule ( VIII) pour obtenir le composé de configuration (E) de formule (Ia) où R₄ et R₅ ne sont pas des atomes d'hydrogène

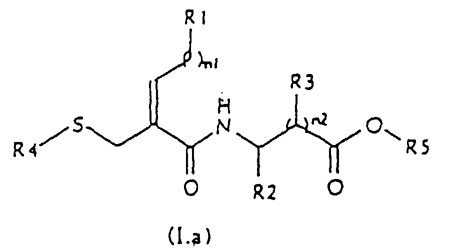

8. Médicament présentant une activité inhibitrice de l'enképhalinase, caractérisé en ce qu'il contient, à titre de principe actif, un composé selon l'une quelconque des revendications 1 à 3.

9. Médicament selon la revendication 8, caractérisé en ce qu'il est présenté sous forme de doses unitaires contenant de 20 à 200 mg de principe actif.

## Patentansprüche

1. Aminosäure-Derivate, dadurch gekennzeichnet, daß sie den folgenden allgemeinen Formeln entsprechen 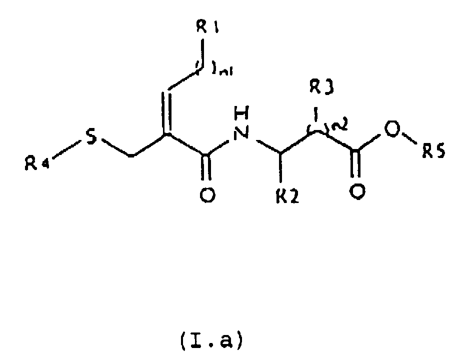 und 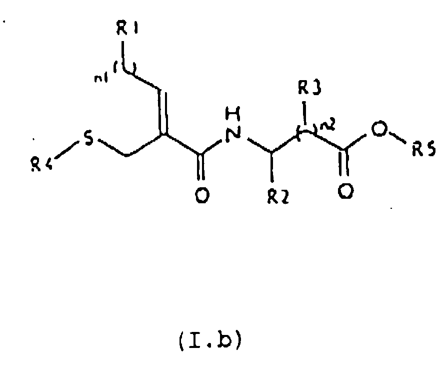 in denen R₁ ein Wasserstoffatom, eine Phenylgruppe, gegebenenfalls einfach oder mehrfach substituiert durch ein Halogenatom, eine Trifluormethylgruppe, eine Nitrogruppe, eine Cyanogruppe oder eine Aminogruppe, eine niedere Alkylgruppe, ein niederes Phenylalkylen, die Gruppe 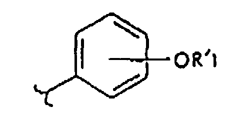 worin R'₁ ein Wasserstoffatom bedeutet, eine niedere Alkylgruppe, eine niedere Phenylalkylengruppe, eine Gruppe 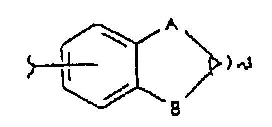 worin A, B und n₃ die folgende Bedeutung haben
| A | B | n₃ |
|---|---|---|
| O | O | 1 |
| O | CH₂ | 1 |
| CH₂ | CH₂ | 1 |
| O | O | 2 |
| CH₂ | CH₂ | 2 |
| O | CH₂ | 2 |
eine Biphenylgruppe, alpha- und beta-Naphthylgruppe, worin
n₁ 0 bis 10 sein kann,
n₂ 1 bis 10 sein kann,
R₂ ein Wasserstoffatom, eine niedere Alkylgruppe, eine niedere Hydroxyalkylengruppe, eine Phenylgruppe, eine niedere Phenylalkylengruppe, eine niedere Hydroxyphenylalkylengruppe, eine niedere Aminoalkylengruppe, eine niedere Guanidinoalkylengruppe, eine niedere Mercaptoalkylengruppe, eine niedere Thioalkylengruppe mit niederem Alkylenrest, eine niedere Imidazolylalkylengruppe, eine niedere Indolylalkylengruppe, eine niedere Carbamoylalkylengruppe, eine niedere Carboxyalkylengruppe bedeutet;
R₃ ebenfalls ein Wasserstoffatom oder eine niedere Alkylgruppe bedeutet;
R₄ ein Wasserstoffatom, einen geradkettigen oder verzweigten aliphatischen Acylrest, einen aromatischen, gegebenenfalls einfach oder mehrfach substituierten aromatischen Acylrest oder einen geradkettigen oder verzweigten Acylrest, der ein oder mehrere Sauerstoffatome aufweist, bedeutet;
R₅ ein Wasserstoffatom, eine niedere geradkettige oder verzweigte Alkylgruppe, eine Phenylgruppe, eine niedere Phenylalkylengruppe, wobei die beiden letzten Gruppen gegebenenfalls einfach oder mehrfach am Phenylkern substituiert sind, einen geradkettigen oder verzweigten Substituenten, der ein oder mehrere Sauerstoffatome besitzt, bedeutet,
wobei die Begriffe "niederes Alkyl" oder "niederes Alkylen" jeweils Alkylgruppen oder Alkylengruppen mit geraden oder verzweigten Ketten mit 1 bis 6 Kohlenstoffatomen, vorzugsweise 1 bis 4 Kohlenstoffatomen bedeuten.

2. Aminosäure-Derivate nach Anspruch 1, dadurch gekennzeichnet, daß R₁ eine Phenylgruppe ist, die gegebenenfalls einfach oder mehrfach substituiert ist mit einem Wasserstoffatom, mit einer Trifluormethylgruppe, der Gruppe 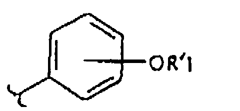 worin R'₁ eine niedere Alkylgruppe, eine Phenylgruppe, die Gruppierung 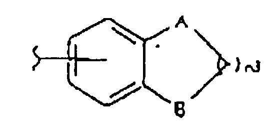 ist, in der A und B Sauerstoff bedeuten und n₃ gleich 1 oder 2 ist, eine Biphenylgruppe, alpha- und beta-Naphthylgruppe;
R₂ und R₃ ein Wasserstoffatom, einen niederen Alkylrest bedeuten;
R₄ ein Wasserstoffatom, einen geradkettigen oder verzweigten aliphatischen Acylrest oder einen aromatischen Acylrest bedeutet;
R₅ ein Wasserstoffatom, einen niederen Alkylrest, einen Phenylrest bedeutet,
n₁ 0 oder 1 ist,
n₂ 1 oder 2 ist,

3. Aminosäure-Derivate nach Anspruch 1, dadurch gekennzeichnet, daß sie ausgewählt sind aus:
- N-(E)-[1-Oxo-2-(acetylthiomethyl)-3-phenylpropenyl]-β-benzylalaninat,
- N-(E)-[1-Oxo-2-(acetylthiomethyl)-3-phenylpropenyl]-β-methylalaninat,
- N-(E)-[1-Oxo-2-(acetylthiomethyl)-3-phenylpropenyl]-β-ethylalaninat,
- N-(E)-[1-Oxo-2-(mercaptomethyl)-3-phenylpropenyl]-β-alanin,
- N- (E)-[1-Oxo-2-(benzoylthiomethyl)-3-phenylpropenyl] -β-benzylalaninat,
- N-(E)-[1-Oxo-2-(benzoylthiomethyl)-3-phenylpropenyl] -β-methylalaninat,
- N-(E)-[1-Oxo-2-(benzoylthiomethyl)-3-phenylpropenyl] -β-ethylalaninat,
- N-(E)-[1-Oxo-2-(pivaloylthiomethyl)-3-phenylpropenyl]-β-benzylalaninat,
- N-(E)-[1-Oxo-2-(pivaloylthiomethyl)-3-phenylpropenyl]-β-methylalaninat,
- N-(E)-[1-Oxo-2-(pivaloylthiomethyl)-3-phenylpropenyl]-β-ethylalaninat,
- N-(E)-[1-oxo-2-(acetylthiomethyl)-3-phenylpropenyl]-(RS)-3-aminomethylbutanoat,
- N-(E)-[1-Oxo-2-(mercaptomethyl)-3-phenylpropenyl]-(RS)-3-aminobutansäure,
- N-(E)-[1-Oxo-2-(acetylthiomethyl)-3-phenylpropenyl]-(RS)-3-amino-2-methyl (methylpropanoat),
- N-(E)-[1-oxo-2-(mercaptomethyl)-3-phenylpropenyl]-(RS)-3-amino-2-methylpropansäure,
- N-(E)-[1-Oxo-2-(acetylthiomethyl)-3-phenylpropenyl]-4-aminobenzylbutanoat,
- N-(E)-[1-Oxo-2-(mercaptomethyl)-3-phenylpropenyl]-4-aminobutansäure,
- N-(Z)-[1-Oxo-2-(acetylthiomethyl)-3-(4-methoxyphenyl) propenyl]-β-benzylalaninat,
- N-(E)-[1-Oxa-2-(acetylthiomethyl)-3-(4-methoxyphenyl)propenyl]-β-benzylalaninat,
- N-(E)-[1-Oxo-2-(mercaptomethyl)-3-(4-methoxyphenyl)propenyl]-β-alanin,
- N-(Z)-[1-Oxo-2-(acetylthiomethyl)-3-(4-trifluormethylphenyl)propenyl]-β-benzylalaninat,
- N-(E)-[1-Oxo-2-(acetylthiomethyl)-3-(4-trifluormethylphenyl)propenyl]-β-benzylalaninat,
- N-(E)-[1-Oxo-2-(mercaptomethyl)-3-(4-trifluormethylphenyl)propenyl]-β-alanin,
- N-(Z)-[1-Oxo-2-(acetylthiomethyl)-3-(4-phenylphenyl)propenyl]-β-benzylalaninat,
- N-(E)-[1-Oxo-2-(acetylthiomethyl)-3-(4-phenylphenyl)propenyl]-β-benzylalaninat,
- N-(E)-[1-Oxo-2-(mercaptomethyl)-3-(4-phenylphenyl)propenyl]-β-alanin,
- N-(Z)-[1-Oxo-2-(acetylthiomethyl)-3-(4-ethoxyphenyl)propenyl]-β-benzylalaninat,
- N-(E)-[1-Oxo-2-(acetylthiomethyl)-3-(4-ethoxyphenyl)propenyl]-β-benzylalaninat,
- N-(E)-[1-Oxo-2-(mercaptomethyl)-3-(4-ethoxyphenyl)propenyl]-β-alanin,
- N-(Z)-[1-Oxo-2-(acetylthiomethyl)-3-(4-propoxyphenyl)propenyl]-β-benzylalaninat,
- N-(E)-[1-Oxo-2-(acetylthiomethyl)-3-(4-propoxyphenyl) propenyl]-β-benzylalaninat,
- N-(Z)-[1-Oxo-2-(acetylthiomethyl)-3-(3,5-difluorphenyl)propenyl]-β-benzylalaninat,
- N-(E)-[1-Oxo-2-(acetylthiomethyl)-3-(3,5-difluorphenyl)propenyl]-β-benzylalaninat,
- N-(E)-[1-Oxo-2-(mercaptomethyl)-3-(3,5-difluorphenyl)propenyl]-β-alanin,
- N-(z)-[1-Oxo-2-(acetylthiomethyl)-3-(1-naphthyl)propenyl]-β-benzylalaninat,
- N-(E)-[1-Oxo-2-(acetylthiomethyl)-3-(1-naphthyl)propenyl]-β-benzylalaninat,
- N-(E)-[1-Oxo-2-(mercaptomethyl)-3-(1-naphthyl)propenyl]-β-alanin,
- N-(Z)-[1-Oxo-2-(acetylthiomethyl)-3-phenylpropenyl]-β-benzylalaninat,
- N-(Z)-[1-oxo-2-(mercaptomethyl)-3-phenylpropenyl]-β-alanin,
- N-(Z)-[1-Oxo-2-(acetylthiomethyl)-3-(4-phenoxyphenyl)propenyl]-β-benzylalaninat,
- N-(E)-[1-Oxo-2-(acetylthiomethyl)-3-(4-phenoxyphenyl)propenyl]-β-benzylalaninat,
- N-(E)-[1-Oxo-2-(mercaptomethyl)-3-(4-phenoxyphenyl)propenyl]-β-alanin,
- N-(Z)-[1-Oxo-2-(acetylthiomethyl)-3-(3,4-methylendioxyphenyl)propenyl]-β-benzylalaninat,
- N-(E)-[1-Oxo-2-(acetylthiomethyl)-3-(3,4-methylendioxyphenyl)propenyl]-β-benzylalaninat,

4. Verfahren zur Herstellung der Aminosäure-Derivate der Formel (Ib), worin R₁, R₂, R₃, R₄, R₅, n₁, n₂ und n₃ die in Anspruch 1 angegebene Bedeutung haben, dadurch gekennzeichnet, daß
a) eine allylische Bromierung einer ethylenischen Säure der Kontiguration (E) der Formel (II) 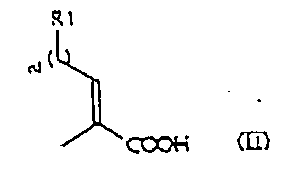 durchgeführt wird, in der R₁ die zuvor angegebene Bedeutung hat, mit einem Bromierungsmittel wie dem N-Bromosuccinimid in Gegenwart einer katalytischen Menge von Benzoylperoxyd, um eine Säure der Formel (III) herzustellen 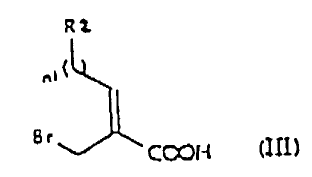
b) Substitution des Broms der Säure der Formel (III), vorzugsweise durch eine Thiosäure R₄-SH zur Bildung eines ethylenischen Thioacyls der Konfiguration (Z) der Formel (IV) 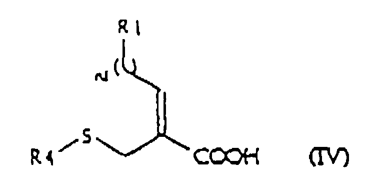 worin R₄ die in Anspruch 1 angegebene Bedeutung hat,
c) Kuppeln der Säure der Formel (IV) mit dem Salz eines Aminosäureesters der Formel (V) 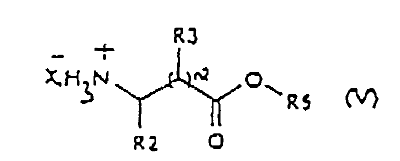 worin R₂, R₃, R₅, und n₂ die in Anspruch 1 angegebenen Bedeutungen haben und X beispielsweise Halogene, Benzolsulfonat, Methansulfonat und Toluolsulfonat bedeutet, zur Bildung der Verbindung der Konfiguration (Z) der Formel (Ib) 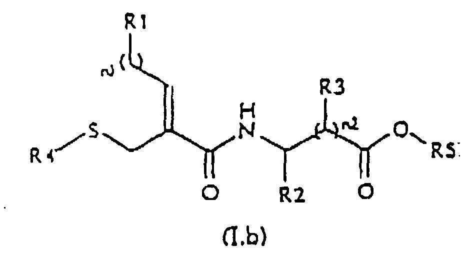 worin R₄ und R₅ keine Wasserstoffatome sind,
d) Unterziehen der Verbindung der Formel (Ib) einer alkalischen Deprotektionierung, um die Verbindungen der Konfiguration (Z) der Formel (Ib) zu bilden, worin R₄ und R₅ Wasserstoffatome sind

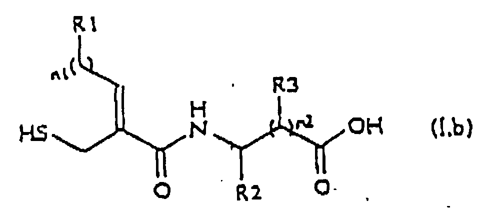

5. Verfahren zur Herstellung von Aminosäure-Derivaten der Formel (Ia), worin R₁, R₂, R₃, R₄, R₅, n₁, n₂ und n₃ die in Anspruch 1 angegebenen Bedeutungen haben, dadurch gekennzeichnet, daß aufeinanderfolgend
a) eine ethylenische Säure der Konfiguration (Z) der Formel (IV) 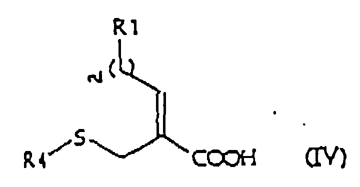 worin R₁, R₄ und n₁ die in Anspruch 1 angegebenen Bedeutungen haben isomerisiert wird, anschließend das erhaltene Isomerengemisch (E/Z) durch ein Amin, wie Cyclohexylamin, getrennt wird, um eine thioacylierte ethylenische Säure der Konfiguration (E) der Formel (VI) 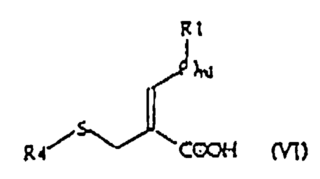 zu erhalten,
b) die Säure der Formel (VI) mit dem gewünschten Salz eines Aminoesters der Formel (V) in Gegenwart eines Kupplungsmittels wie Dicyclohexylcarbodiimid gekuppelt wird, um die Verbindung der Formel (Ia) der Konfiguration (E) zu erhalten, worin R₄ und R₅ keine Wasserstoffatome sind, 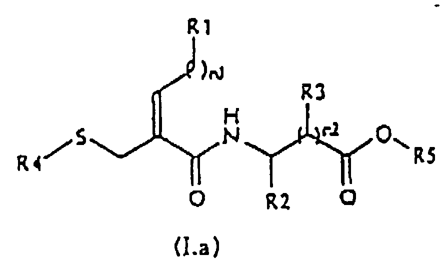
c) die Verbindung der Formel (Ia) anschließend einer alkalischen Deprotektionierung unterzogen wird, um die Verbindungen der Konfiguration (E) der Formel (Ia) zu erhalten, worin R₄ und R₅ Wasserstoffatome sind.

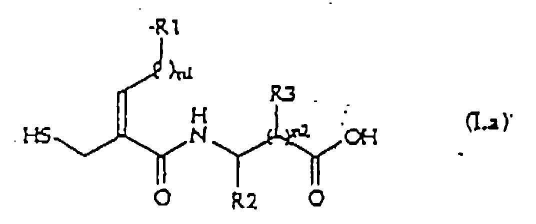

6. Verfahren zur Herstellung von Aminosäure-Derivaten der Formel (Ia), worin R₁, R₂, R₃, R₄, R₅, n₁, n₂ und n₃ die in Anspruch angegebenen Bedeutungen haben dadurch gekennzeichnet, daß aufeinanderfolgend
a) eine ethylenische Säure der Formel (IV), die die Konfiguration (Z) besitzt, isomerisiert wird, um eine thioacylierte ethylenische Säure in Form eines Isomerengemischs der Konfiguration (Z/E) der Formel (VII) 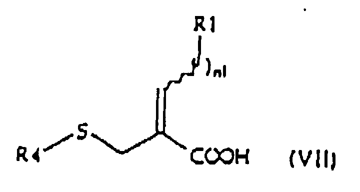 zu erhalten, worin R₁, R₄ und n₁ die in Anspruch 1 angegebenen Bedeutungen haben,
b) die Säure der Formel (VII) mit dem Salz des gewünschten Aminoesters der Formel (V) in Gegenwart eines Kupplungsmittel wie Dicyclohexylcarbodiimid gekuppelt wird, um eine Verbindung der Formel (VIII) zu erhalten, die eine Mischung der Isomeren (Z/E) bildet 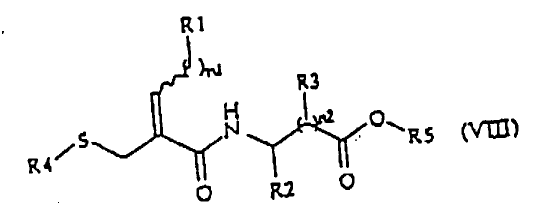
c) das Isomerengemisch (Z/E) der Verbindung der Formel (VIII), beispielsweise durch eine Flashchromatagraphie über Siliciumoxid und durch fraktionierte Rekristallisation getrennt wird, um die Verbindung der Konfiguration (E) der Formel (Ia) zu erhalten, worin R₄ und R₅ keine Wasserstoffatome sind

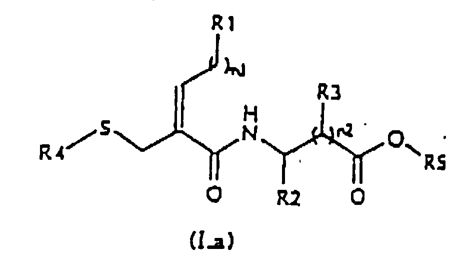

7. Verfahren zur Herstellung von Aminosäure-Derivaten der Formel (Ia), worin R₁, R₂, R₃, R₄, R₅, n₁, n₂ und n₃ die in Anspruch 1 angegebenen Bedeutungen haben, R₄ und R₅ keine Wasserstoffatome sind, dadurch gekennzeichnet, daß man aufeinanderfolgend
a) die Verbindung der Formel (Ib), die eine Doppelbindung der Konfiguration (Z) aufweist 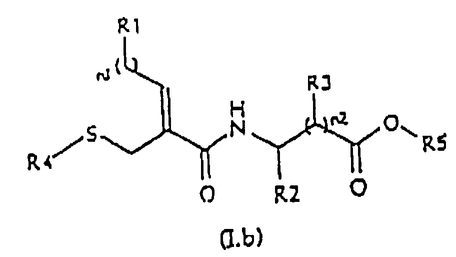 vorzugsweise in Gegenwart eines Bortrifluoretherats isomerisiert, um eine Verbindung der Formel (VIII), die aus einem Isomerengemisch (Z/E) besteht, zu erhalten. 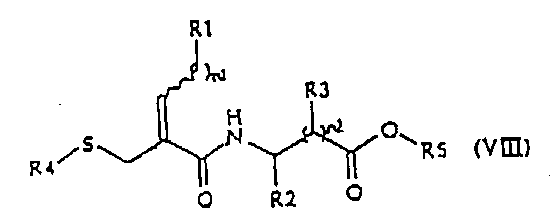
b) das Isomerengemisch (Z/E) der Verbindung der Formel (VIII) getrennt wird, um eine Verbindung der Konfiguration (E) der Formel (Ia) zu erhalten, worin R₄ und R₅ keine Wasserstoffatome sind

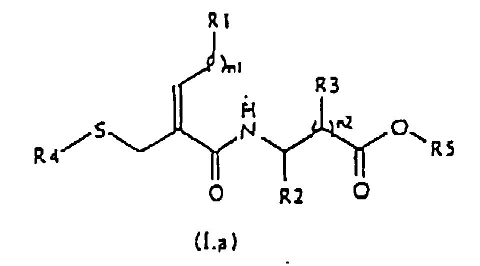

8. Arzneimittel mit einer inhibierenden Wirkung auf die Enkephalinase, dadurch gekennzeichnet, daß es als wirksame Verbindung eine Verbindung nach einem der Ansprüche 1 bis 3 enthält.

9. Arzneimittel nach Anspruch 8, dadurch gekennzeichnet, daß es in Einzeldosisform, die 20 bis 200 mg des wirksamen Mittels enthält, vorliegt.

## Claims

1. Amino acid derivatives, characterised in that they correspond to the general formulae 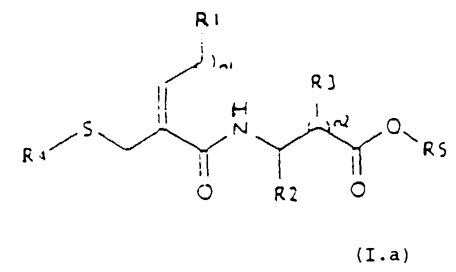 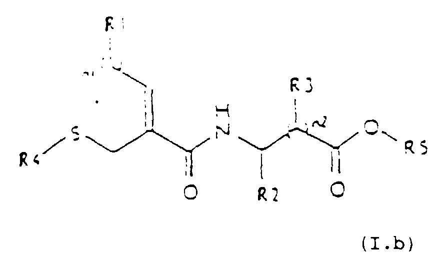 in which R₁ represents a hydrogen atom, a phenyl group optionally mono- or polysubstituted with a halogen atom, a trifluoromethyl group, a nitro group, a cyano group or an amino group, a lower alkyl group, a lower phenylalkylene group; the grouping 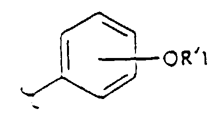 where R'₁ represents a hydrogen atom, a lower alkyl group, a phenyl group, a lower phenylalkylene group; a grouping 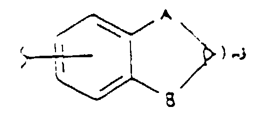 where A, B and n₃ denote the following
| A | B | n₃ |
|---|---|---|
| 0 | 0 | 1 |
| 0 | CH₂ | 1 |
| CH₂ | CH₂ | 1 |
| 0 | 0 | 2 |
| CH₂ | CH₂ | 2 |
| 0 | CH₂ | 2 |
a biphenyl, alpha and beta naphthyl group,
n₁ varies from 0 to 10,
n₂ varies from 1 to 10,
R₂ represents a hydrogen atom, a lower alkyl group, a lower hydroxyalkylene group, a phenyl group, a lower phenylalkylene group, a lower hydroxyphenylalkylene group, a lower aminoalkylene group, a lower guanidinoalkylene group, a lower mercaptoalkylene group, a lower thioalkylene-lower alkyl group, a lower imidazolylalkylene group, a lower indolylalkylene group, a lower carbamoylalkylene group, a lower carboxyalkylene group;
R₃ likewise represents a hydrogen atom or a lower alkyl group;
R₄ represents a hydrogen atom, a straight or branched-chain aliphatic acyl radical, an optionally mono- or polysubstituted aromatic acyl radical or a straight or branched-chain acyl radical containing one or more oxygen atoms;
R₅ represents a hydrogen atom, a straight or branched-chain lower alkyl group, a phenyl group, a lower phenylalkylene group, with the latter two groups being optionally mono- or polysubstituted in the phenyl ring, a straight or branched-chain substituent containing one or more oxygen atoms,
with the terms "lower alkyl" or "lower alkylene" denoting respectively a straight or branched-chain alkyl or alkylene group having 1 to 6 carbon atoms, preferably 1 to 4 carbon atoms.

2. Amino acid derivatives according to Claim 1, characterised in that R₁ represents a phenyl group which is optionally mono- or polysubstituted with a halogen atom, or with the trifluoromethyl group; the grouping 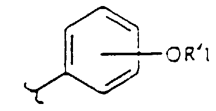 where R'₁ represents a lower alkyl group, a phenyl group; the grouping 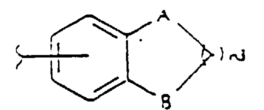 where A and B represent oxygen, and n₃ is equal to 1 or 2; a biphenyl, alpha and beta naphthyl group;
R₂ and R₃ represent a hydrogen atom, a lower alkyl radical;
R₄ represents a hydrogen atom, a straight or branched-chain aliphatic acyl radical or an aromatic acyl radical;
R₅ represents a hydrogen atom, a lower alkyl radical, a phenyl radical;
n₁ is equal to 0 or 1;
n₂ is equal to 1 or 2.

3. Amino acid derivatives according to Claim 1, characterised in that they are selected from among:
- N-(E)-[1-oxo 2-(acetylthiomethyl) 3-phenyl propenyl]-β-alanine benzyl ester,
- N-(E)-[1-oxo 2-(acetylthiomethyl) 3-phenyl propenyl]-β-alanine methyl ester,
- N-(E)-[1-oxo 2-(acetylthiomethyl) 3-phenyl propenyl]-β-alanine ethyl ester,
- N-(E)-[1-oxo 2-(mercaptomethyl) 3-phenyl propenyl]-β-alanine,
- N-(E)-[1-oxo 2-(benzoylthiomethyl) 3-phenyl propenyl]-β-alanine benzyl ester,
- N-(E)-[1-oxo 2-(benzoylthiomethyl) 3-phenyl propenyl]-β-alanine methyl ester,
- N-(E)-[1-oxo 2-(benzoylthiomethyl) 3-phenyl propenyl]-β-alanine ethyl ester,
- N-(E)-[1-oxo 2-(pivaloylthiomethyl) 3-phenyl propenyl]-β-alanine benzyl ester,
- N-(E)-[1-oxo 2-(pivaloylthiomethyl) 3-phenyl propenyl]-β-alanine methyl ester,
- N-(E)-[1-oxo 2-(pivaloylthiomethyl) 3-phenyl propenyl]-β-alanine ethyl ester,
- N-(E)-[1-oxo 2-(acetylthiomethyl) 3-phenyl propenyl]-(RS)-3-aminobutyric acid methyl ester,
- N-(E)-[1-oxo 2-(mercaptomethyl) 3-phenyl propenyl]-(RS)-3-aminobutyric acid,
- N-(E)-[1-oxo 2-(acetylthiomethyl) 3-phenyl propenyl]-(RS)-3-amino-2-methylpropionic acid methyl ester,
- N-(E)-[1-oxo 2-(mercaptomethyl) 3-phenyl propenyl]-(RS)-3-amino-2-methylpropionic acid,
- N-(E)-[1-oxo 2-(acetylthiomethyl) 3-phenyl propenyl]-4-aminobutyric acid benzyl ester,
- N-(E)-[1-oxo 2-(mercaptomethyl) 3-phenyl propenyl]-4-aminobutyric acid,
- N-(Z)-[1-oxo 2-(acetylthiomethyl) 3-(4-methoxyphenyl) propenyl]-β-alanine benzyl ester,
- N-(E)-[1-oxo 2-(acetylthiomethyl) 3-(4-methoxyphenyl) propenyl]-β-alanine benzyl ester,
- N-(E)-[1-oxo 2-(mercaptomethyl) 3-(4-methoxyphenyl) propenyl]-β-alanine,
- N-(Z)-[1-oxo 2-(acetylthiomethyl) 3-(4-trifluoromethyl phenyl) propenyl]-β-alanine benzyl ester,
- N-(E)-[1-oxo 2-(acetylthiomethyl) 3-(4-trifluoromethyl phenyl) propenyl]-β-alanine benzyl ester,
- N-(E)-[1-oxo 2-(mercaptomethyl) 3-(4-trifluoromethyl phenyl) propenyl]-β-alanine,
- N-(Z)-[1-oxo 2-(acetylthiomethyl) 3-(4-phenyl phenyl) propenyl]-β-alanine benzyl ester,
- N-(E)-[1-oxo 2-(acetylthiomethyl) 3-(4-phenyl phenyl) propenyl]-β-alanine benzyl ester,
- N-(E)-[1-oxo 2-(mercaptomethyl) 3-(4-phenyl phenyl) propenyl]-β-alanine,
- N-(Z)-[1-oxo 2-(acetylthiomethyl) 3-(4-ethoxy phenyl) propenyl]-β-alanine benzyl ester,
- N-(E)-[1-oxo 2-(acetylthiomethyl) 3-(4-ethoxy phenyl) propenyl]-β-alanine benzyl ester,
- N-(E)-[1-oxo 2-(mercaptomethyl) 3-(4-ethoxy phenyl) propenyl]-β-alanine,
- N-(Z)-[1-oxo 2-(acetylthiomethyl) 3-(4-propoxy phenyl) propenyl]-β-alanine benzyl ester,
- N-(E)-[1-oxo 2-(acetylthiomethyl) 3-(4-propoxy phenyl) propenyl]-β-alanine benzyl ester,
- N-(Z)-[1-oxo 2-(acetylthiomethyl) 3-(3,5-difluoro phenyl) propenyl]-β-alanine benzyl ester,
- N-(E)-[1-oxo 2-(acetylthiomethyl) 3-(3,5-difluoro phenyl) propenyl]-β-alanine benzyl ester,
- N-(E)-[1-oxo 2-(mercaptomethyl) 3-(3,5-difluoro phenyl) propenyl]-β-alanine,
- N-(Z)-[1-oxo 2-(acetylthiomethyl) 3-(1-naphthyl) propenyl]-β-alanine benzyl ester,
- N-(E)-[1-oxo 2-(acetylthiomethyl) 3-(1-naphthyl) propenyl]-β-alanine benzyl ester,
- N-(E)-[1-oxo 2-(mercaptomethyl) 3-(1-naphthyl) propenyl]-β-alanine,
- N-(Z)-[1-oxo 2-(acetylthiomethyl) 3-phenyl propenyl]-β-alanine benzyl ester,
- N-(Z)-[1-oxo 2-(mercaptomethyl) 3-phenyl propenyl]-β-alanine,
- N-(Z)-[1-oxo 2-(acetylthiomethyl) 3-(4-phenoxy phenyl) propenyl]-β-alanine benzyl ester,
- N-(E)-[1-oxo 2-(acetylthiomethyl) 3-(4-phenoxy phenyl) propenyl]-β-alanine benzyl ester,
- N-(E)-[1-oxo 2-(mercaptomethyl) 3-(4-phenoxy phenyl) propenyl]-β-alanine,
- N-(Z)-[1-oxo 2-(acetylthiomethyl) 3-(3,4-methylene dioxyphenyl) propenyl]-β-alanine benzyl ester,
- N-(E)-[1-oxo 2-(acetylthiomethyl) 3-(3,4-methylene dioxyphenyl) propenyl]-β-alanine benzyl ester.

4. A process for the preparation of the amino acid derivatives of the formula (Ib) in which R₁, R₂, R₃, R₄, R₅, n₁, n₂ and n₃ denote the same as indicated in Claim 1, characterised in that it comprises successively:
a) carrying out an allylic bromination of an ethylenic acid of the configuration (E) of the formula (II) 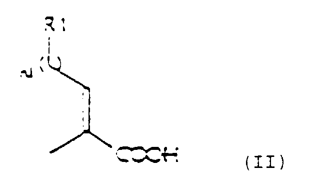 in which R₁ denotes the same as above, using a brominating agent such as N-bromosuccinimide in the presence of a catalytic quantity of benzoyl peroxide, in order to form an acid of the formula (III) 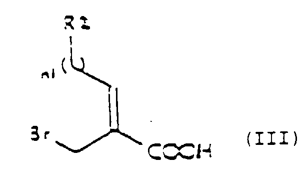
b) substituting the bromine of the acid of the formula (III), preferably with an R₄-SH thioacid, in order to form the thioacylated ethylenic acid of the configuration (Z) of the formula (IV) 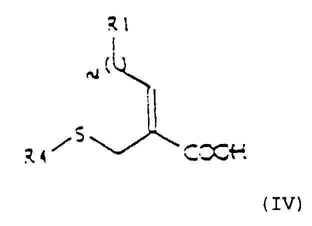 where R₄ has the definition indicated in Claim 1,
c) coupling the acid of the formula (IV) with the salt of the desired amino ester of the formula (V) 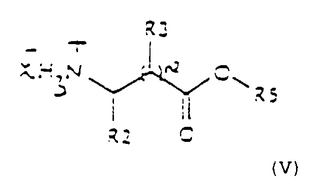 where R₂, R₃, R₅ and n₂ denote the same as indicated in Claim 1, and X represents, for example, halides, benzene sulphonate, methane sulphonate and toluene sulphonate, in order to form the compound of the configuration (Z) of the formula (Ib) 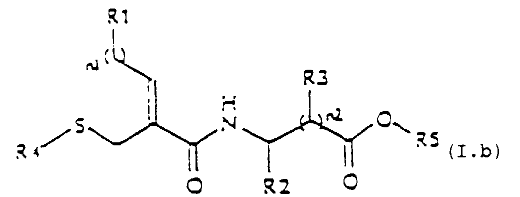 where R₄ and R₅ are not hydrogen atoms,
d) removing by alkaline means the protection of the compound of the formula (Ib), in order to form the compounds of the configuration (Z) of the formula (Ib) where R₄ and R₅ are hydrogen atoms

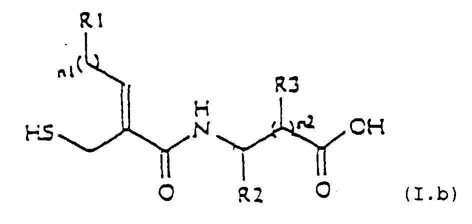

5. A process for the preparation of the amino acid derivatives of the formula (Ia) where R₁, R₂, R₃, R₄, R₅, n₁, n₂ and n₃ denote the same as indicated in Claim 1, characterised in that it comprises successively:
a) isomerising the ethylenic acid of the configuration (Z) of the formula (IV) 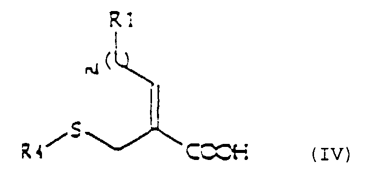 where R₁, R₄ and n₁ denote the same as indicated in Claim 1, then separating the (E/Z) isomer mixture obtained, using an amine such as cyclohexylamine, in order to obtain the thioacylated ethylenic acid of the configuration (E) of the formula (VI) 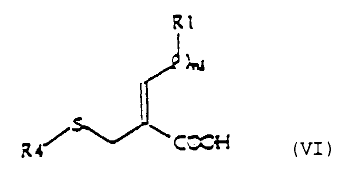
b) coupling the acid of the formula (VI) with the salt of the desired amino ester of the formula (V), in the presence of a coupling agent such as dicyclohexyl carbodiimide, in order to obtain the compound of the formula (Ia) of the configuration (E) where R₄ and R₅ are not hydrogen atoms 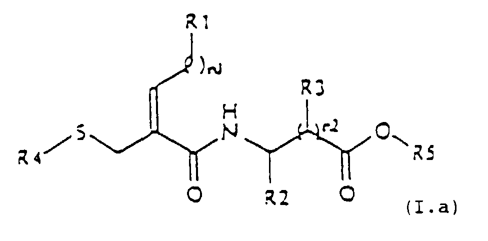
c) then removing by alkaline means the protection of the compound of the formula (Ia), in order to obtain the compounds of the configuration (E) of the formula (Ia) where R₄ and R₅ are hydrogen atoms

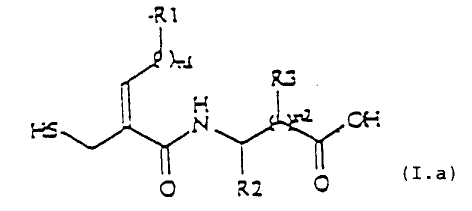

6. A process for the preparation of the amino acid derivatives of the formula (Ia) where R₁, R₂, R₃, R₄, R₅, n₁, n₂ and n₃ denote the same as indicated in Claim 1, characterised in that it comprises successively:
a) isomerising the ethylenic acid of the formula (IV) which has a (Z) configuration, in order to obtain the thioacylated ethylenic acid in the form of a mixture of isomers of the configuration (Z/E) of the formula (VII) 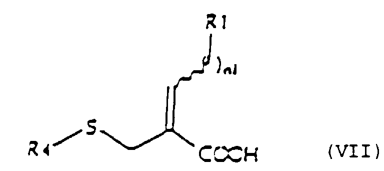 where R₁, R₄ and n₁ denote the same as indicated in Claim 1,
b) coupling the acid of the formula (VII) with the salt of the desired amino ester of the formula (V), in the presence of a coupling agent such as dicyclohexyl carbodiimide, in order to obtain the compound of the formula (VIII) which is constituted by a (Z/E) isomer mixture 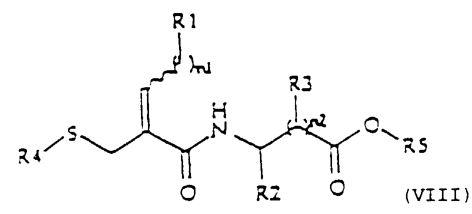
c) separating the (Z/E) isomer mixture of the compound of the formula (VIII), for example by flash chromatography with silica or by fractional recrystallisation, in order to obtain the compound of the configuration (E) of the formula (Ia) where R₄ and R₅ are not hydrogen atoms

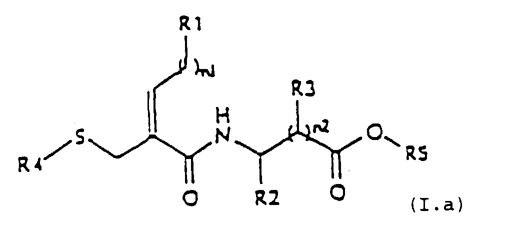

7. A process for the preparation of the amino acid derivatives of the formula (Ia) where R₁, R₂, R₃, R₄, R₅, n₁, n₂ and n₃ denote the same as indicated in Claim 1, with R₄ and R₅ not being hydrogen atoms, characterised in that it comprises successively:
a) isomerising the compound of the formula (Ib) which has a double bond and the configuration (Z) 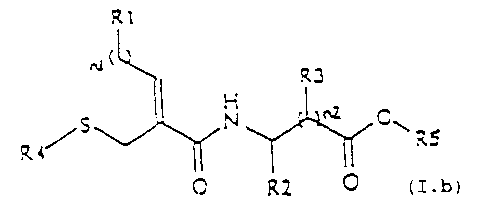 preferably in the presence of boron trifluoride etherate, in order to obtain the compound of the formula (VIII) which is constituted by a (Z/E) isomer mixture 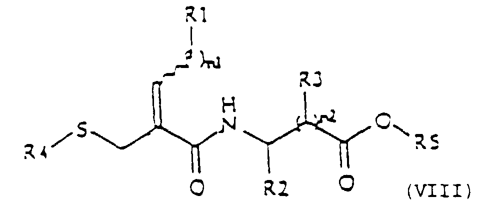
b) separating the (Z/E) isomer mixture of the compound of the formula (VIII) in order to obtain the compound of the configuration (E) of the formula (Ia) where R₄ and R₅ are not hydrogen atoms

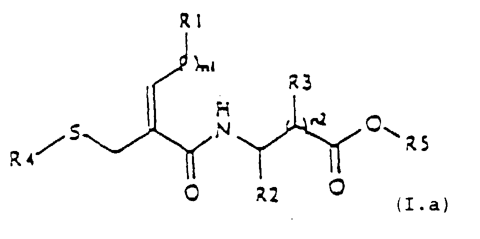

8. A medicament having an encephalinase-inhibiting activity, characterised in that it contains as the active principle a compound according to any one of Claims 1 to 3.

9. A medicament according to Claim 8, characterised in that it is presented in the form of unit doses containing from 20 to 200 mg of active principle.
